# EUROPEAN PATENT SPECIFICATION

(11) **EP 3 532 110 B1**
(45) Date of publication and mention of the grant of the patent: **07.06.2023**
(21) Application number: 17790836.5
(22) Date of filing: 20.10.2017
(51) Int. Cl.: A61L 26/00, A61F 13/00, A61K 33/00, A61L 15/18, A61L 15/32, A61L 15/44, A61Q 19/00, A61Q 19/08, A61K 9/06, A61K 47/34, A61P 17/00

(54) **FORMULATIONS FOR OXYGEN DELIVERY TO BODY TISSUES**
FORMULIERUNGEN ZUR SAUERSTOFFABGABE AN KÖRPERGEWEBE
FORMULATION POUR L'APPORT D'OXYGÈNE À DES TISSUS CORPORELS

(30) Priority: 26.10.2016 GB 201618110; 08.09.2017 GB 201714461
(43) Date of publication of application: 04.09.2019
(73) Proprietor: Oxy Solutions AS, 0349 Oslo (NO)
(72) Inventor: MOEN, Ingrid, 0564 Oslo (NO); UGLAND, Hege, 1368 Stabekk (NO); AMIRY-MOGHADDAM, Mahmood, 1358 Jar (NO); HAGLERØD, Camilla, 1550 Hølen (NO)
(74) Representative: Dehns
(86) International application number: PCT/GB2017/053185
(87) International publication number: WO 2018/078340

(56) References cited:
- WO-A1-98/53836
- WO-A1-2010/135449
- WO-A1-2016/071691
- GB-A- 2 087 882
- US-A1- 2012 269 899
- US-B2- 7 160 553

## Description

### Technical field

The present invention relates generally to oxygen delivery to body tissues, in particular to compromised tissues, including wounds, burns, inflamed and/or infected tissues (for example those associated with acne and other inflammatory skin disorders).

More specifically, the invention relates to topical formulations capable of delivering high levels of dissolved oxygen directly to compromised tissues in order to aid in healing and/or regeneration. It further relates to methods for the preparation of such formulations, to delivery devices (such as wound dressings, bandages, etc.) which incorporate these, and to their use as medicaments in the topical treatment of compromised tissues.

The invention also relates to the use of such topical formulations in methods of cosmetic treatment of body surfaces, especially the skin.

### Background of the invention

Any tissue or cells of a living organism that are not in a normal metabolic state may be considered "compromised".

Inflamed and/or infected tissues may be considered to be compromised. Most skin conditions and diseases of the skin are associated with inflammation. This includes conditions such as acne, in particular acne vulgaris which is a chronic inflammatory disease of the pilosebaceous unit. Infection is a complication of many skin disorders in which the skin's barrier is broken, e.g. in conditions such as eczema and non-healing wounds, and this can also lead to inflammation.

Damage to the blood supply to any living tissue rapidly leads to compromised tissue which is no longer in a normal metabolic state. One of the functions of an adequate blood supply is the delivery of dissolved oxygen to body tissues. Poor oxygen delivery results in slow healing of damaged tissues, the possibility of infection, development of scar tissue and, in some cases, can ultimately lead to tissue death and the need to amputate affected limbs.

Wounds are accompanied by damage or destruction of the blood supply to skin tissues which compromises the delivery of oxygen and nutrients required for tissue regeneration. Healing of wounds is a particular problem in the elderly population. Oxygen, in particular, plays a crucial role in wound healing, including reduction in bacterial infections, increased re-epithelialization, proliferation of fibroblasts, collagen synthesis and angiogenesis. Insufficient oxygenation of wounds due to poor blood circulation thus impairs proper wound healing and can result in the formation of chronic wounds.

Many chronic wounds will typically contain colonies of aerobic and/or anaerobic organisms as part of a biofilm. In 60-100% of chronically open wounds, a biofilm will be present. Bacterial biofilms are common and form when bacteria interact with a body surface to form polymeric films (also known as "exopolysaccharide" or "extracellular polysaccharide" polymers) that coat the body surface and provide a living colony for further bacterial colonisation and proliferation. Bacteria which become lodged in a biofilm are more difficult to remove or kill than those that remain in a plaktonic state (i.e. suspended as single cells) and can be resistant to many antibiotics. Studies have suggested that oxygen may play a role in the reduction of biofilm formation.

Wound care currently accounts for about 4% of total healthcare costs worldwide and is expected to grow significantly with the ageing population. One approach to the treatment of wounds is to increase the oxygen tension in the environment of the damaged tissue either by systemic administration of oxygen to the patient and/or by localised, topical delivery.

Oxygen gas therapies in which oxygen gas is delivered to a patient is well established for use in the treatment of chronic wounds. In some cases, wound tissues may be oxygenated directly by diffusion. Alternatively, oxygen gas dissolves in the blood thereby increasing blood oxygen levels, which in turn enhances the delivery of oxygen to the body tissues. Raising oxygen levels in the wound tissue aids in the healing process.

Currently, the most commonly used oxygen-based therapy for wound healing is HBOT (Hyperbaric Oxygen Therapy). This involves placing the patient in a pressure chamber and the treatment is based on exposure and breathing pure oxygen gas which is delivered at a pressure greater than ambient pressure. However, this treatment requires specialized equipment and highly skilled personnel which results in a high cost to the healthcare system. The treatment also severely reduces the patient's mobility whilst undergoing the treatment. Other disadvantages of HBOT are that it is time-consuming and uncomfortable for the patient - many patients experience claustrophobia and barotrauma during the treatment - and that the treatment may result in poor transfer of oxygen from the oxygen-rich atmosphere to the hypoxic tissue at the wound site (due to disrupted blood circulation) whilst also increasing the chances of oxygen toxicity and resulting organ damage.

Alternatives to HBOT include Topical Oxygen Therapy (TOT) and Continuous Diffusion of Oxygen (CDO) which involve localised diffusion of oxygen gas directly into the wound site, either continuously or for a specific treatment period. TOT is achieved via a sleeve that encases the patient's limb, which is supplied with oxygen gas and pressurized slightly more than atmospheric pressure. However, there is controversy as to the depth of absorption of topical oxygen and therefore its efficacy. CDO provides continuous, sustained oxygen therapy. CDO therapy uses a disposable oxygen concentrator and cannula together with a wound dressing to continuously supply the wound with oxygen. The patient is free to ambulate while being treated 24 hours a day.

Other approaches to wound treatment include the use of oxygenated dressings. These are inexpensive options for oxygen therapy, however, the number of products currently on the market is limited. These either incorporate oxygen predominantly in the form of oxygen gas bubbles or contain components which generate oxygen gas when in use. Examples of such products include the OxyBand^{™}, OxygeneSys^{™} and Oxyzyme^{™} dressings.

The OxyBand^{™} dressing (OxyBand Technologies, MN, USA) provides for the local delivery of high concentrations of pure oxygen to healing wounds using a directionally permeable, gas-emitting reservoir. The oxygen is stored in a reservoir inbetween an occlusive upper layer and a lower oxygen-permeable film which allows the dressing to supersaturate the wound fluid with oxygen (Lairet et al., J. Burn Care Res. 35(3): 214-8, 2014; Lairet et al., abstract at The Military Health Services Research Symposium, 2012; and Hopf et al., abstract of the Undersea & Hyperbaric Medical Society Annual Scientific Meeting, 2008).

The OxygeneSys^{™} dressing comprises a polyacrylate matrix that forms a closed cell foam structure encapsulating oxygen gas. The walls of the foam cells of the matrix contain dissolved oxygen. When the dressing is moistened with exudate, saline or water the gaseous oxygen within the dressing begins to dissolve into the liquid, but the release rate of oxygen is low and only reaches 15 mg/L (see e.g. US patent No. 7,160,553).

Oxyzyme^{™} is an enzyme-activated hydrogel dressing system which comprises two polysulphonate sheet hydrogels layered on top of one another. Also contained within the dressing are an oxidase enzyme, glucose and iodide. When removed from its packaging and contacted with a wound, the oxidase enzyme within the top layer is activated upon contact with oxygen in the air and by the contact made between the two layers of the dressing. Reaction of the enzyme with oxygen generates hydrogen peroxide within the dressing which, when it reaches the wound-facing surface, is converted through its interaction with the iodine component of the dressing into dissolved oxygen (Ivins et al., Wounds UK, Vol. 3 No. 1, 2007; and Lafferty et al., Wounds UK, Vol. 7 No. 1, 2011).

Oxygenated dressings represent an improvement in the delivery of topical oxygen to the wound environment over the hyperbaric chamber and have shown encouraging results in case studies (see, for example, Lairet *et al*., 2014; Lairet *et al*., 2012; Hopf *et al*., 2008; Ivins *et al*., 2007; and Lafferty *et al*., 2011 (all as above); Roe et al., Journal of Surgical Research 159: e29-e36, 2010; Zellner et al., Journal of International Medical Research Vol. 43(1), 93-103, 2014; and Kellar et al., Journal of Cosmetic Dermatology 12: 86-95, 2013). However, documentation of the oxygen concentration/availability and oxygen stability of these currently available products is limited and these are not in widespread use.

Recent studies have shown that dissolved oxygen diffuses and penetrates tissue more efficiently compared to directly exposing the tissue to oxygen gas (see e.g. Roe *et al*., 2010 (as above), Stüker, J. Physiol. 538(3): 985-994, 2002; Atrux-Tallau et al., Skin Pharmacol. Physiol. 22: 210-217, 2009; Reading et al., Int. J. Cosmetic Sci. 35:600-603, 2013; and Charton et al., Drug Design, Devel. and Ther. 8:1161-1167, 2014). None of the existing therapies enables the direct delivery of high levels of dissolved oxygen to compromised tissues. For the most part, these deliver oxygen in the form of a gas which must dissolve (e.g. in wound exudate or other cellular fluid) before it can be effective. This limits the efficacy of the treatment. Although the OxygeneSys^{™} dressing contains some dissolved oxygen in the moisture which coats the walls of the foam matrix, the release rate of oxygen only reaches a maximum of 15 mg/L. A treatment which provides a high level of oxygen directly to the tissues in dissolved form would thus be beneficial.

A need thus exists for alternative means for the delivery of oxygen to treat compromised tissues. In particular, a need exists for a cost-effective treatment with minimal inconvenience to the patient in which oxygen can be delivered to compromised tissues, including inflamed tissues, in dissolved form thereby improving the healing rate of conditions such as inflammatory acne and wounds, especially chronic skin wounds. The present invention addresses these needs.

### Summary of the invention

The present invention provides an alternative method of delivering oxygen to compromised body tissues, e.g. to inflamed tissues or to a wound site, to aid in healing, regeneration or restoration of a normal metabolic state. It further provides a method of delivering oxygen to a body surface of a patient (e.g. to the surface of the skin) in order to improve or otherwise enhance its appearance.

In at least some embodiments, the invention provides an improved method for the delivery of oxygen in which high and stable levels of oxygen are supplied to body tissues in the form of dissolved oxygen which is able to penetrate more rapidly into the tissues than oxygen gas.

Specifically, the present inventors have developed novel topical formulations which contain high levels of dissolved oxygen. At the point of use these are provided in the form of a "liquid" (which includes thickened or 'viscous' liquids) which is convenient to apply to the target tissue irrespective of its size and location. Following administration, these display an increase in viscosity and are transformed into a gel which conforms to the geometry of the target tissue and which is capable of the release of dissolved oxygen directly at the point of contact with the compromised tissues. Such formulations are referred to herein as *"in-situ* gelling".

The formulations may be applied directly to the body tissues, or these may be incorporated into a suitable delivery means (herein a "delivery device") which is intended to be applied to the desired area of the body. For example, the formulations may be provided in, or as a component of, a dressing, bandage or other wound covering which is suitable for application to the target site.

In one aspect the invention thus provides a liquid formulation comprising at least 20 mg/L dissolved oxygen and an *in-situ* gelling agent which is capable of forming a gel upon contact with a body surface or body tissues and which comprises one or more poloxamers, and wherein said gel is capable of releasing a therapeutically effective amount of said dissolved oxygen.

In another aspect the invention provides an oxygen-releasing gel obtainable by allowing the liquid formulation herein described to gel.

In a further aspect the invention provides a formulation or oxygen-releasing gel as herein described for use in medicine or for use as a medicament.

In another aspect the invention provides a kit for use in the treatment of a compromised body surface or body tissue of a mammalian subject (e.g. a human), the kit comprising:
(a) a sealed container or package containing a liquid formulation as herein described; and
(b) a delivery device, e.g. a wound dressing or bandage.

The kit may additionally comprise instructions for use of the components of the kit in the topical treatment of a compromised tissue, e.g. instructions for its use according to any method as herein described.

In another aspect, the invention provides a delivery device (e.g. a wound dressing or bandage) having incorporated therein a liquid formulation as herein described.

In a further aspect, the invention provides a method of cosmetic treatment performed on a mammalian subject (e.g. a human), said method comprising the step of administering to the surface of the skin of said subject a liquid formulation as herein described.

In one embodiment, the present invention provides formulations capable of delivering stable, controlled and high levels of dissolved oxygen to compromised tissues thereby improving the rate of tissue healing at a lower cost than the most commonly used oxygen therapies available in the market.

In one embodiment, the liquid formulations herein described are capable of forming a gel having an oxygen stability for a prolonged period of time, for example at least 30 hours, when applied to skin or other body tissues, for example when these are covered by a dressing or film at the point of use.

The products and methods herein described are suitable for home care thus reducing healthcare costs, e.g. avoiding the need for long term hospitalization, whilst also allowing patients full mobility during treatment.

### Detailed description of the invention

### Definitions:

Unless otherwise defined, the term "liquid" as used herein refers to a substance which flows freely and which maintains a constant volume. It includes thickened liquids and viscous liquids which flow. A "liquid" will typically have a loss modulus (G") which is greater than its storage modulus (G') and a loss tangent (tan δ) which is greater than 1.

As used herein, the term "gel" refers to a substance which is self-holding yet deformable, i.e. which is not a solid. Typically, a "gel" will have a loss modulus (G") which is less than its storage modulus (G') and a loss tangent (tan δ) which is less than 1.

Storage modulus (or `elastic modulus'), G', represents the elastic nature of a material. Loss modulus (or `viscous modulus'), G", represents the viscoelastic nature of a material. For a sol-gel material the loss tangent (tan δ) is a measure of the ratio of the energy lost to the energy stored. The formation of a `gel' occurs when G" is equal to G' and tan δ is equal to 1.

As used herein "thermogelling" refers to a formulation which is generally liquid at ambient (room) temperature in the range of about 20 to 25°C but which gels at higher temperatures, e.g. at body temperature in the range of from about 30 to about 40°C, preferably about 34 to about 37°C, e.g. at about 34 to about 35°C. Preferably the formulation undergoes the transition from liquid to a gel in the range from about 30 to about 37°C.

The "sol-gel transition temperature" is the temperature at which a thermogelling formulation undergoes the transition from a liquid to a gel.

The term "delivery device" as used herein means any device intended to be applied to a body tissue or body surface and which is intended to remain in place to deliver dissolved oxygen to the body tissues. It encompasses materials such as wound dressings, wound coverings, bandages, patches, plasters, etc. As will be described, in some embodiments the invention relates to such a delivery device which contains an oxygenated liquid formulation as herein described.

A "compromised tissue" includes any tissue or cells of a living organism that are not in a normal metabolic state. It includes inflamed and/or infected tissues and any tissue having a reduced or interrupted blood supply, such as that caused by hypoxic conditions, occlusions, blockages, surgery, a degenerative process or a traumatic injury. The tissue or cells need not be in a living body at the point of treatment, for example these may be tissues or cells which are awaiting transplantation, or which are growing in a culture dish.

A "wound" includes any defect or disruption in the skin which may result from physical, chemical or thermal damage, or as a result of an underlying medical or physiological condition. Wounds may be classified as acute or chronic wounds.

A "bacterial biofilm" means a community of bacteria which are contained within an extracellular polymeric substance (EPS) matrix produced by the bacteria and attached to a body surface.

As used herein, the term "cosmetic" is intended to define a formulation or treatment method which is intended solely for cosmetic purposes, e.g. to enhance, improve or otherwise maintain the appearance of the subject (e.g. a human subject) to which the formulation is applied.

The formulations according to the invention are provided in the form of a liquid which is conveniently applied to the desired target site. The liquid makes intimate contact with the target tissues and once in place sets or hardens to form a gel which remains at the target site, maintains conformity with the tissues and can deliver the active oxygen in a controlled manner. The gel also serves to moisturise the target tissues. The resulting gel has a higher viscosity than the liquid formulation from which it was formed. The gel is self-holding and no longer flows.

The liquid formulation forms a gel at the target site due to the presence therein of at least one *in-situ* gelling agent. Once set, the gel may take a number of different forms depending on the nature of the underlying tissues, for example this may form a block, a sheet or film which covers the tissues to be treated.

The *in-situ* gelling formulations are thermogelling. These exhibit an increase in viscosity and form a cohesive, viscous `gel' at higher temperatures, preferably at body temperature.

In the context of the invention it is suitable that the thermogelling formulations may have a storage modulus (G') at ambient temperature of below about 150 Pa, more suitably below about 100 Pa, and preferably as low as about 0.01 to 50 Pa. The storage modulus at body temperature (i.e. once gelled) may typically be in the range of 5,000 to 30,000 Pa, preferably 8,000 to 20,000 Pa, more preferably 15,000 to 19,000 Pa, e.g. about 17,000 to 18,000 Pa.

The thermogelling agent comprises poloxamers which are non-ionic block copolymers of polyoxy(ethylene) and polyoxy(propylene). These may be represented by the following general formula in which "a" represents the number of hydrophilic ethylene oxide chains and "b" represents the number of hydrophobic propylene oxide chains:

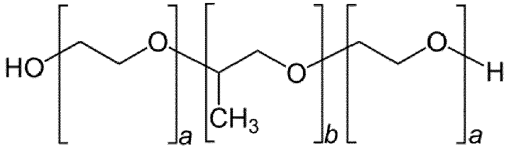

In this formula, "a" is an integer in the range from 12 to 101, and "b" is an integer in the range from 20 to 56.

Poloxamers are commercially available in different grades which vary from liquids to solids. By varying the values of "a" and "b" other properties of the poloxamers can also be adjusted as desired, e.g. gelling temperature (T_{sol-gel}), storage modulus (G'), degree of hydrophobicity, etc. Their properties can also be adjusted by blending different poloxamers having different molecular weights.

Suitable poloxamers and poloxamer blends for use in the invention may be selected by those skilled in the art. Those having a thermogelling temperature above about 25°C, e.g. in the range from 25 to 37°C may be used in the invention.

Examples of suitable poloxamers include Poloxamer 407, 188, 338, 124, 237, and mixtures thereof. These are commercially available from a number of suppliers, such as BASF under the tradename Pluronic^{®}, or from Sigma-Aldrich under the tradename Kolliphor^{®}.

Preferred for use in the invention is Poloxamer 407 (Pluronic^{®} F 127) in which "a" is 95-105 and "b" is 54-60 (at the maximum of its molecular weight distribution, "a" is about 101 and "b" is about 56). This particular poloxamer thermogels in the range from 25 to 40°C.

Other preferred poloxamers include Poloxamer 188 (Pluronic^{®} F68) (in which "a" is 75-85 and "b" is 25-40 - at the maximum of its molecular weight distribution, "a" is 80 and "b" is 27), Poloxamer 338 (Pluronic^{®} F108) (in which "a" is 137-146 and "b" is 42-47), and Poloxamer 124 (Pluronic^{®} L44) (in which "a" is 10-15 and "b" is 18-23).

Suitable poloxamer blends may comprise a higher molecular weight poloxamer and a lower molecular weight poloxamer, and preferably will include the higher molecular weight poloxamer in excess to the lower molecular weight poloxamer. In one embodiment the blends may include a combination of Poloxamer 407 with Poloxamer 188. In this case, Poloxamer 188 may be added to increase gelling and to improve viscosity of the formulation at ambient temperature. An alternative blend is that formed from Poloxamer 407 and Poloxamer 124. In this case, the addition of the liquid Poloxamer 124 improves processability of the formulation under ambient temperature conditions. Other suitable combinations may readily be determined by those skilled in the art.

Suitable blends of poloxamers and the ratio of the poloxamer components may be selected according to the desired characteristics of the blend, e.g. the desired gelling temperature. Blends which comprise two poloxamers having different molecular weights may be used. Where two poloxamers are present, the higher molecular weight poloxamer will generally form the major component. For example, two poloxamers may be present in a blend in a ratio (higher molecular weight poloxamer: lower molecular weight poloxamer) from 5:1 to 50:1, preferably from 10:1 to 25:1, e.g. from 10:1 to 15:1. A preferred blend is that containing Poloxamer 407 and Poloxamer 188 in a ratio of about 18.5:1.5.

Suitable concentrations of poloxamer may be determined by those skilled in the art taking into account the other components present in the formulation and the requirement that this should form a gel *in-situ.* Typically, the poloxamer (or blend of poloxamers) will be present in the liquid formulation in an amount of at least about 15 wt.% (based on the total weight of the formulation). For example, the poloxamer concentration may be in the range of from 10 to 30 wt.%, preferably 15 to 25 wt.%. e.g. 16 to 20 wt.%. The poloxamer concentration will affect Tsol-gel (this decreases with an increase in poloxamer concentration) and this can be adjusted accordingly.

Other components may be present in the thermogelling formulations to aid in gelling. These include thickening agents such as Carbopol, celluose derivatives (e.g. hydroxypropyl cellulose or hydroxypropyl methylcellulose), carrageenans, gelatin, pectin, hydrocolloids, alginates, hydrogels, polyurethane, collagen, chitosan, and hyaluronic acid. As would generally be understood, when any additional thickening agent is present, the concentration of poloxamer required for preparation of an *in-situ* gel forming system may be lower. The presence of any additional gelling agent is optional.

In one embodiment, the formulations according to the invention may additionally include a hydrogel. A hydrogel is a network of insoluble, hydrophilic polymers that can swell in water and hold a large amount of water while maintaining the structure. A three-dimensional network is formed by cross-linking of polymer chains, e.g. by covalent bonds, hydrogen bonding, van der Waals forces or ionic bonds. Suitable hydrogel materials are well known in the pharmaceutical industry and may be readily selected by those skilled in the art. These include both natural and synthetic polymer materials. Examples of suitable materials include, but are not limited to, the following: microcrystalline cellulose, cellulose and cellulose derivatives (e.g. ethyl cellulose, methyl cellulose, carboxymethyl cellulose, hydroxypropylmethyl cellulose, hydroxypropyl cellulose), gums (e.g. acacia gum, tragacanth gum), gelatin, carrageenans, alginates (e.g. sodium alginate), pectin, carbomers, modified starch, poly(methacrylates), and polyvinylpyrrolidine.

In one embodiment, the hydrogel materials may be a polysaccharide, including chitosans, dextran, hyaluronic acid, agars, alginates, starch, cellulose and cellulose derivatives, glycogen, carrageenans, galactomannans and combinations thereof.

Hydrogels are particularly suitable for the treatment of dry, sloughy or necrotic wounds by rehydrating dead tissues and enhancing autolytic debridement. They also serve to promote moist healing, are non-adherent and cool the surface of the wound.

Other components which may be present in the thermogelling formulations include bioadhesives, e.g. mucoahesive polymers.

The formulations of the invention contain dissolved, molecular oxygen and are capable of releasing this to the target tissues following *in-situ* gelling. Since this is intended to function as an active and to deliver a certain level of oxygen to the tissues, its concentration should be chosen accordingly. The precise oxygen level will depend on various factors, including the precise nature of the formulation (e.g. the other components which may be present and their stability in the presence of oxygen), the intended use and duration of any treatment, the patient to whom the formulation is to be administered, etc. Suitable levels may readily be determined by those skilled in the art according to need.

The formulations comprise at least 20 mg/L dissolved oxygen, preferably up to 100 mg/L, e.g. from 20 to 100 mg/L, preferably from 20 to 70 mg/L, from 20 to 60 mg/L, or from 25 to 50 mg/L. Formulations comprising elevated levels of oxygen, for example at least 25 mg/L or least 30 mg/L, are particularly preferred. In one set of embodiments, dissolved oxygen levels may range from 30 to 55 mg/L, e.g. from 35 to 50 mg/L, or from 40 to 50 mg/L.

The wound healing process involves various overlapping stages in which a variety of cellular and matrix components act together to re-establish integrity of damaged tissue and replacement of lost tissue. These are generally considered to involve: haemostasis, inflammation, migration, proliferation and maturation phases. Acute hypoxia stimulates angiogenesis, whereas raised tissue oxygen levels stimulate epithelialisation and fibroblasts. Different concentrations of oxygen may be employed during the different stages of wound healing.

An important aspect of the invention is that the formulations may contain a high and stable level of dissolved oxygen. In this context, by "stable" it is intended that the formulations should maintain their oxygen level for a period of at least 2 weeks, preferably at least 6 months when stored under suitable storage conditions, e.g. at a temperature in the range of from 4 to 25°C, preferably when stored at lower temperatures, e.g. in the range from 2 to 4°C. A further aspect of the invention is that the formulations are capable of maintaining a high and stable level of dissolved oxygen once applied to the target tissues, e.g. following application to the skin and, optionally, following occlusion (e.g. by application of a suitable wound covering). Preferably, "stable" levels of dissolved oxygen are maintained for a period of at least 4 hours, preferably at least 30 hours following application.

The oxygen present in the formulations will be dissolved in an aqueous medium which is physiologically tolerable, for example a physiological salt solution (e.g. saline) or water. Typically this will be a physiological salt solution.

Liquids, such as water, containing dissolved oxygen and methods for their preparation are generally known in the prior art. Any of these liquids may be employed in preparing the formulations herein described. Particularly preferred for use in the invention are those liquids having an increased oxygen concentration and which have increased retention rates of oxygen within the liquid over time. Examples of such liquids are those described in WO 02/26367 and WO 2010/077962. Liquids having a high level of oxygen for use in preparation of the formulations according to the present invention may conveniently be produced according to the methods and by means of the devices described therein. The liquids (e.g. water) having a high level of oxygen used to produce the formulations of the invention may be produced according to the methods which are described in these earlier applications. Such methods involve the following steps:
- introducing a pressurized liquid into a piping network to form a flow stream,
- if necessary, adding colloidal minerals to a desired concentration to the flow stream,
- injecting gaseous oxygen into the flow stream to produce a mixture of liquid and oxygen bubbles,
- providing a linear flow accelerator including a venturi and a magnet adjacent to the venturi and aligned along the venturi for applying a magnetic field of desired field strength across the venturi, and
- passing the flowing mixture of liquid and gaseous oxygen bubbles through the linear flow accelerator to accelerate the flowing mixture and to subsequently decelerate the flowing mixture to subsonic speed to break up the gaseous oxygen bubbles.

Oxygenation using the above described method makes it possible to produce an oxygenated liquid (e.g. water) having a high and stable oxygen content. When the liquid is water, the solubility of oxygen is increased from about 7 mg/L to 50, 60, 70 mg/l or more, and the oxygen content is substantially stable in a cooled bottle for months.

As a result of the processes described in these earlier documents for the super-oxygenation of liquids, the oxygenated liquid comprises an amount of colloidal minerals, for example from 10 to 50 ppm colloidal minerals. By "colloidal mineral" is meant an insoluble inorganic particulate which remains suspended in the liquid in which it is present, i.e. a material which remains substantially non-dissolved in the liquids used according to the present invention, especially a material which is insoluble in water. Colloidal minerals typically have an average particle size (e.g. a mean average particle diameter) of from 1 to 1000 nm, especially between 2 and 200 nm, e.g. less than 10 nm. Methods for determining the sizes of nanoparticles are well known and include dynamic light scattering, laser diffraction and microscopy, e.g. scanning electron microscopy and atomic force microscopy.

Colloidal minerals are characterized by having electrostatic adsorption of ions to the surface of a colloidal particle. This adsorption creates a primary adsorption layer that in turn creates a substantial adsorption layer at the surface of the colloidal particle. This surface charge performs two functions: (1) the surface charge causes a repulsion to exist between two particles when they approach each other, and (2) the surface charge attracts oppositely charged ions into the vicinity of the particles. As a result, an ion "cloud" or "double layer" forms in a solution around the charged particles and the ions are dispersed throughout the liquid. Thus, the colloidal minerals provide electrostatic surface ion absorption characteristics that can enhance the ability of the liquid (e.g. water) in which they are present to absorb and retain oxygen. The amount and types of colloidal minerals which may be present in the liquid (e.g. water) for use in preparing the formulations herein described will depend on the requirements of any given application, for example, the desired oxygen content.

Examples of suitable colloidal minerals include aluminium, sulphur, iron and fluoride. Specific mineral compositions that may be used include those produced by The Rockland Corporation (Tulsa, Oklahoma, USA) under the trademark Body Booster, and by TRC Nutritional Laboratories, Inc. (Tulsa, Oklahoma, USA) under the trademark TRC Minerals^{®}. A suitable formulation of 77LPPM TRC Minerals is set out in the Table of FIG. 2 of WO 2010/077962.

The liquid having a high level of oxygen produced as indicated above is stable and may be stored in tanks or be bottled between production and use in preparation of the formulations according to the present invention.

In an alternative embodiment, the oxygenated liquid which is used to prepare the formulations of the invention may be produced using a method and apparatus which is a development of that disclosed in WO 02/26367 and WO 2010/077962 in which no colloidal minerals are employed and no magnetic field is applied. Such an apparatus comprises:
a liquid inlet for supplying liquid (e.g. water) into the apparatus;
an oxygen inlet for supplying oxygen into the liquid within the apparatus to create a liquid and oxygen mixture, the oxygen inlet being in fluid communication with, and downstream of, the liquid inlet;
a venturi in fluid communication with, and downstream of, the liquid inlet and the oxygen inlet, wherein the venturi is arranged to dissolve the oxygen into the liquid passing through the venturi; and
an outlet for the oxygenated liquid (e.g. water) in fluid communication with, and downstream of, the venturi.

This apparatus comprises liquid and oxygen inlets and an outlet, with a venturi therebetween. Liquid and oxygen are supplied into the apparatus via the respective inlets, the oxygen inlet being positioned downstream of the liquid inlet such that the oxygen is injected into the liquid stream. This liquid and oxygen mixture is then passed to a venturi, e.g. via a conduit in fluid communication with, and downstream of, the liquid inlet and the oxygen inlet, the conduit being arranged to supply the liquid and the oxygen to the venturi. Owing to the restriction the venturi creates in the flow path, this causes the liquid and oxygen mixture to accelerate through the venturi and then decelerate at the other side, generating a shockwave in the liquid and oxygen mixture which forces the oxygen to dissolve in the liquid, thus oxygenating the liquid.

The apparatus may comprise a mixing chamber in fluid communication with, and downstream of, the oxygen inlet and the liquid inlet (and also the diffusion chamber in the embodiment in which it is provided), the mixing chamber being arranged to induce turbulence into the fluid flowing therethrough. The mixing chamber produces turbulent flow of the liquid and the oxygen flowing through the mixing chamber which acts to break-up the oxygen into small bubbles within the liquid so that they are more easily dissolved into the liquid in the mixing chamber and downstream in the apparatus, e.g. in the venturi. The mixing chamber may be provided in any suitable and desired way, i.e. to induce the necessary turbulent flow. For example, the mixing chamber may comprise one or more obstacles (e.g. barriers in the flow path) and/or a tortuous path.

When using this apparatus the oxygen and liquid (e.g. water) mixture passing through the venturi need not be exposed to a magnetic field, and the apparatus is arranged such that the liquid (e.g. water) and oxygen mixture that is supplied to the venturi contains substantially no colloidal minerals. By "substantially no colloidal minerals" it is intended that, if present, these are in negligible amounts, e.g. less than 50 ppm, preferably less than 20 ppm, more preferably less than 10 ppm.

An alternative method for producing the oxygenated liquid (e.g. water) for use in the invention may thus comprise the following steps:
- introducing a pressurized liquid (e.g. water) into a piping network to form a flow stream;
- injecting gaseous oxygen into the flow stream to produce a mixture of liquid and oxygen bubbles,
- providing a linear flow accelerator including a venturi; and
- passing the flowing mixture of liquid and gaseous oxygen bubbles through the linear flow accelerator to accelerate the flowing mixture and to subsequently decelerate the flowing mixture to subsonic speed to break up the gaseous oxygen bubbles.

Methods of oxygenating a liquid which do not involve the use of colloidal minerals or a magnet are also described in WO 2016/071691.

Any of the apparatus herein described may be used with any liquid as is suitable and desired. In this context, the term "liquid" includes liquids in the conventional sense as well as materials which are flowable, e.g. a thickened or viscous liquid, or a flowable gel. Although typically it will be a physiological salt solution or water which is oxygenated and used to produce the formulations of the invention, any other liquid or otherwise 'flowable' component or components of the formulations may be oxygenated prior to admixture with the remaining components. Alternatively, depending on the precise nature and components of the final formulations, these may be oxygenated using any of the apparatus and methods described herein in their final form.

Thus, in one set of embodiments, the oxygenated formulations may be prepared by oxygenation of a suitable liquid formulation comprising a substance which is capable of forming a gel as herein described. For example, these may be produced by a method comprising the following steps:
- introducing a liquid (e.g. water) which comprises one or more *in-situ* gelling agents (i.e. one or more poloxamers) into a piping network to form a flow stream;
- injecting gaseous oxygen into the flow stream to produce a mixture of said liquid and oxygen bubbles; and
- passing the flowing mixture of liquid and gaseous oxygen bubbles through a venturi which is arranged to dissolve the gas into the liquid passing through the venturi.

In this method, the liquid introduced into the piping network to form the flow stream may be pressurised, but it need not be. Typically, it will not be pressurised at the point of introduction into the piping network.

Any of the methods herein described for preparation of the oxygenated liquids or the oxygenated formulations may further comprise the step of introducing the liquid (which may or may not contain the *in-situ* gelling agents) into a holding volume (e.g. a holding tank) as described in WO 2016/071691. The liquid may be introduced into the holding volume prior to the formation of the liquid and oxygen mixture, or it may be introduced into the holding volume downstream of the venturi. The holding volume may be pressurised, but it need not be. The liquid in the holding tank may, if required, be agitated to maintain the homogeneity of the liquid.

In any of the methods herein described, the liquid for oxygenation may further contain one or more foam reducing agents (e.g. simethicone), or the methods may comprise an additional foam reducing step. The foam reducing step may comprise any suitable and desired method and it may be provided at any suitable point in the oxygenation method. In one embodiment, the foam reducing step may comprise introduction of the liquid into a holding volume (e.g. a holding tank) as herein described.

The apparatus herein described is capable of producing oxygenated liquid (e.g. physiological salt solution or water) with a concentration of dissolved oxygen of greater than 20 mg/L, e.g. greater than 40 mg/L, e.g. greater than 50 mg/L, e.g. greater than 60 mg/L, e.g. approximately 70 mg/L. Oxygenation levels up to about 100 mg/L, e.g. up to about 90 mg/L or up to 80 mg/L, may be achieved. Such oxygen levels can also be achieved when using the apparatus to oxygenate a liquid containing one or more *in-situ* gelling agents (i.e. one or more poloxamers) as herein described.

In the treatment of compromised tissues or cells it may be beneficial to deliver other active agents to the site of injury. In one embodiment, at least one other active substance may also be present in the formulations, for example a combination of other active substances. These include substances known to be suitable for the treatment of compromised tissues, such as inflamed tissues, wounds, burns, etc.

Other active agents which may be present in any of the formulations herein described include antibacterial agents, antifungal agents, antiviral agents, antibiotics, growth factors, cytokines, chemokines (e.g. macrophage chemo-attractant protein (MCP-1 or CCL2), nucleic acids, including DNA, RNA, siRNA, micro RNA, vitamins (e.g. vitamins A, C, E, B), minerals (e.g. zinc, copper, magnesium, iron, silver, gold), anaesthetics (e.g. benzocaine, lidocaine, pramoxine, dibucaine, prilocaine, phenol, hydrocortisone), anti-inflammatory agents (e.g. corticosteroids, iodide solutions), moisturizers (e.g. hyaluronic acid, urea, lactic acid, lactate and glycolic acid), extracellular matrix proteins (e.g collagen, hyaluronan, and elastin), enzymes (e.g. enzymes in the hatching fluid from fish roe, or in roe extracts such as salmon egg extract), stem cells from plants, extracts from eggs and eggshells (e.g. from salmon and hen's eggs), botanical extracts, fatty acids (e.g. omega-6 and omega-3 fatty acids, in particular polyunsaturated fatty acids), and skin penetration enhancers.

Growth factors exert potent and critical influence on normal wound healing. Wound repair is controlled by growth factors (platelet-derived growth factor [PDGF], keratinocyte growth factor, and transforming growth factor-β). PDGF is important for most phases of wound healing. Recombinant human variants of PDGF-BB (Becaplermin) have been successfully applied in diabetic and pressure ulcers. Growth factors which may be provide in the formulations include epidermal growth factor (EGF), platelet derived growth factor (PDGF), fibroblast growth factor (FGF), keratinocyte growth factor (KGF or FGF 7), vascular endothelial growth factor (VEGF), transforming growth factor (TGF-b 1), insulin-like growth factor (IGF-1), human growth hormone and granulocyte-macrophage colony stimulating factor (GM-CSF).

Cytokines, e.g. the interleukin (IL) family and tumor necrosis factor-α family promote healing by various pathways, such as stimulating the production of components of the basement membrane, preventing dehydration, increasing inflammation and the formation of granulation tissue. IL-6 is produced by neutrophils and monocytes and has been shown to be important in initiating the healing response. It has a mitogenic and proliferative effect on keratinocytes and is chemoattractive to neutrophils. Examples of cytokines which may be present include the interleukin (IL) family, and tumor necrosis factor-α family.

Vitamins C (L-ascorbic acid), A (retinol), and E (tocopherol) show potent anti-oxidant and anti-inflammatory effects. Vitamin C deficiencies result in impaired healing, and have been linked to decreased collagen synthesis and fibroblast proliferation, decreased angiogenesis, increased capillary fragility, impaired immune response and increased susceptibility to wound infection. Similarly, vitamin A deficiency leads to impaired wound healing. The biological properties of vitamin A include anti-oxidant activity, increased fibroblast proliferation, modulation of cellular differentiation and proliferation, increased collagen and hyaluronate synthesis, and decreased MMP-mediated extracellular matrix degradation.

Several minerals have been shown to be important for optimal wound repair. Magnesium functions as a co-factor for many enzymes involved in protein and collagen synthesis, while copper is a required co-factor for cytochrome oxidase, for cytosolic anti-oxidant superoxide dismutase, and for the optimal cross-linking of collagen. Zinc is a co-factor for both RNA and DNA polymerase, and a zinc deficiency causes a significant impairment in wound healing. Iron is required for the hydroxylation of proline and lysine, and, as a result, severe iron deficiency can result in impaired collagen production.

Where the formulations are intended for use in the treatment of skin conditions these may contain agents known to be suitable for the treatment of such conditions. Where the condition is acne, the additional agents may include retinoids such as vitamin A, acitretin, isotretinion, tretinion and tazarotene; peroxides such as benzoyl peroxide; antibiotics such as tetracycline, clindamycin, erythromycin, metronidazole, sulfacetamide, doxycycline, oxytetracycline, minocycline, and trimethoprim; hormones such as co-cyprindiol, azelaic acid and derivatives thereof; adapalene; nicotinamide; and salicylic acid.

For the treatment of psoriasis, additional active agents may include salicylic acid, corticosteroids, vitamin D and derivatives thereof, retinoids, antralin (inhibits DNA replication), and calcineurin inhibitors.

Collagen plays a vital role in the natural wound healing process from the induction of clotting to the formation and final appearance of the final scar. It stimulates formation of fibroblasts and accelerates the migration of endothelial cells upon contact with damaged tissue. Chitosan accelerates granulation during the proliferative stage and wound healing.

Examples of anti-bacterial agents that may be present include, but are not limited to, the following: alcohols, chlorine, peroxides, aldehydes, triclosan, triclocarban, benzalkonium chloride, linezolid, quinupristin-dalfopristin, daptomycin, oritavancin and dalbavancin, quinolones, moxifloxacin.

Cosmetic formulations may also comprise other active ingredients generally known and used in cosmetics, such as peptides, amino acids, hyaluronic acid, hydroxy acids, vitamins or derivatives thereof, retinoids, ceramides, carbamide (urea) and coenzyme Q10.

The amount of any other active substances may readily be determined by those skilled in the art depending on the choice of active. Typically, this may be in the range from 1 to 10 wt.%, e.g. 1 to 5 wt.% (based on the total weight of the formulation). In the case of cosmetic formulations, these may contain high concentrations of active substances, for example up to 70 wt.%, e.g. up to 30 wt.% (based on the total weight of the formulation).

The formulations herein described are aqueous, but need not be purely aqueous.

The liquid formulations may comprise up to 99 wt.% water. Typically, these will comprise at least 50 wt.% water, more preferably at least 60 wt.% water, yet more preferably at least 70 wt.% water, e.g. at least 80 wt.% water. For example, the liquid formulations herein described may contain from 80 to 99 wt.% water. A relatively high water content ensures a high oxygen level and thus may lead to rapid absorption of the dissolved oxygen into the skin.

One or more lipid or oil carriers may also be present in the formulations according to the invention, for example where these may be required to solubilise any lipid soluble components (e.g. other actives) which may be present. Suitable lipid carriers are well known and used in dermal formulations and any known carriers may be employed. Examples of these include, but are not limited to, fats, waxes, oils, free fatty acids or esters thereof, and fatty alcohols, for example propylene glycol, isolanolin, glycerin, DMSO, glycerol, etc. Where at least one lipid carrier is present, this may be provided in an amount in the range of from 1 to 50 wt.%, preferably 2 to 40 wt.%, e.g. from 3 to 35 wt.%.

In one embodiment the formulations may be provided in the form of an emulsion, for example an oil-in-water or water-in-oil emulsion. Preferred emulsions are oil-in-water emulsions, in particular those comprising more than 50 wt.% water.

The emulsions will typically comprise one or more emulsifiers. These may be selected from any of the emulsifiers generally known and used in formulations to be applied to the skin, particularly the human skin. Such emulsifiers include non-ionic, cationic and anionic compounds. The emulsifiers may be present in the formulations in the range of from 0.1 to 30 wt%, preferably from 0.5 to 20 wt%, e.g. from 1 to 15 wt%.

Where the compositions comprise two or more phases, the aqueous phase will have an oxygen concentration as herein described.

In one embodiment, the formulations may be substantially free from any lipid materials, for example these will contain less than 5 wt.%, e.g. less than 1 wt.% lipid. The formulations may, in another embodiment, contain no lipid carrier and thus be lipid-free.

The formulations according to the invention may comprise other optional components, e.g. components which maintain a buffered pH, or those which maintain osmolality in a range suitable for the intended application, or which maintain stability of the composition. Other components which may be present thus include buffers, pH adjusting agents, osmolality adjusting agents, preservatives (e.g. anti-microbial agents), anti-oxidants, gel forming agents such as Carbopols and cellulose derivatives, fragrances, coloring agents, etc.

The presence of a buffer serves to adjust the pH to physiological levels, e.g. in the range from 3 to 9, preferably from 4 to 7, e.g. about 5.5. A suitable choice of buffer can also aid in controlling the ionic strength of the formulations. Examples of buffers which may be employed include citrate, phosphate, carbonate, and acetate. Isotonic aqueous buffers, such as phosphate, are particularly preferred. Examples of suitable buffers include TRIS, PBS, HEPES.

Wounds with an alkaline pH have lower healing rates than those with a pH closer to neutral. Some studies have also shown that an acidic environment in the wound supports the natural healing process and controls microbial infections. Chronic wounds typically have an elevated alkaline environment and may, for example, have a pH in the range of 7.15 to 8.9. In the treatment of wounds, especially chronic wounds, an acidic pH may thus be advantageous. In one embodiment, the formulations may therefore be buffered to have a pH in the range from 2 to 7. For example, these may be buffered to a pH in the range from 3 to 6.5, preferably from 5 to 6, more preferably from 5 to 5.5, e.g. about 5.1 to about 5.5.
pH adjusting agents which may be present include sodium hydroxide, hydrochloric acid, acetic acid, boric acid, ascorbic acid, hyaluronic acid, and citric acid. In some cases, honey may also be used to adjust the pH.

Salts may also be present in order to adjust the osmolality of the formulation and thus enhance its tolerability *in vivo.* Any suitable salt known in the art for adjusting osmolality may be employed. Osmolality may be adjusted depending on the nature of the wound. For example those with excessive exudate may benefit from a hypertonic gel, whereas for others a hypotonic or isotonic gel may be more appropriate. One example of a suitable salt is sodium chloride. This may be added in an amount ranging from about 0.05 to about 2 wt.%, e.g. about 0.2 to about 1 wt.% (based on the total weight of the formulation) to form an isotonic gel. Higher or lower amounts may be added as required to obtain a hypotonic or hypertonic gel. The presence of sodium chloride further serves to strengthen the resulting gel, to increase its bioadhesive force and increase Tsol-gel.

The choice of any additional components should take into account any negative impact it may have on gel strength of the formulation once gelled. Agents which may reduce the strength of the gel should thus either be used sparingly or not at all.

Suitable preservatives which may be present include, but are not limited to, benzalkonium chloride, sodium chloride, parabens, vitamin E, disodium EDTA, glycerin, ethanol.

The presence of one or more antioxidants may serve to extend the shelf life of the formulations herein described, for example where these may contain any other components which are sensitive to oxidation. Examples of suitable antioxidants which may be present include ascorbic acid and ascorbic acid salts (e.g. sodium ascorbate, potassium ascorbate and calcium ascorbate); fatty acid esters of ascorbic acid such as ascorbyl palmitate and ascorbyl stearate; tocopherols such as alpha-tocopherol, gamma-tocopherol and delta-tocopherol; propyl gallate, octyl gallate, dodecyl gallate or ethyl gallate; guaiac resin; erythorbic acid, sodium erythorbate, erythorbin acid or sodium erthorbin; tert-butylquinone (TBHQ); butylated hydroxyanisole (BHA); butylated hydroxytoluene (BHT); anoxomer and ethoxyquin. Preferred for use in the invention are those antioxidants which are water-soluble such as, for example, ascorbic acid and ascorbate salts.

The optimum amount of antioxidant(s) in the formulations of the invention will depend on a number of factors including the type of formulation, its oxygen level, the presence and amount of any oxygen-sensitive compounds in the formulation, etc. Suitable levels may readily be determined by those skilled in the art. However, the level of antioxidant will typically be at least 0.001 % by weight, especially at least 0.01 or at least 0.03 wt%. The level of antioxidant will typically be less than 5 wt%, especially less than 2 or 1 wt%, e.g. between 0.02 and 0.5 wt% or between 0.05 and 0.2 wt%.

Skin penetration enhancers may also be present and these may have a beneficial effect in enhancing the activity of the formulations. Any of the skin penetration enhancing agents known and described in the pharmaceutical literature may be used. These may include, but are not limited to, any of the following: fatty acids (e.g. oleic acid), dialkyl sulphoxides (such as dimethylsulphoxide, DMSO), Azones (e.g. laurocapram), pyrrolidones and derivatives (e.g. 2-pyrrolidone, 2P), alcohols and alkanols (e.g. ethanol, decanol, isopropanol), glycols (e.g. propylene glycol), and surfactants (e.g. dodecyl sulphate). Examples of other skin penetration enhancing agents include propylene glycol laurate, propylene glycol monolaurate, propylene glycol monocaprylate, isopropyl myristate, sodium lauryl sulphate, dodecyl pyridinium chloride, oleic acid, propylene glycol, diethylene glycol monoethyl ether, nicotinic acid esters, hydrogenated soya phospholipids, essential oils, alcohols (such as ethanol, isopropanol, n-octanol and decanol) , terpenes, N methyl-2-pyrrolidine, polyethylene glycol succinate (TPGS), Tween 80 and other surfactants, and dimethyl-betacyclodextrin. Where present, any surface penetration enhancing agents may be provided in an amount in the range of from 0.1 to 10 wt%, e.g. about 5 wt%.

When used for cosmetic purposes, the formulations should exhibit a pleasant fragrance, appearance and texture (e.g. consistency). Such formulations may therefore also include coloring agents and/or fragrances.

In an embodiment, the liquid formulations according to the invention consist essentially of water, oxygen, a thermogelling agent as herein described, a buffer, an osmolality adjusting agent, and optionally one or more pharmaceutically acceptable carriers or excipients. As used herein, the term "consisting essentially of" means that the formulations do not comprise any other components which materially affect their properties when in use, such as other pharmaceutically acceptable agents which may typically be used in wound treatment.

The formulations herein described may be produced by simple mixing of the various components in the desired amounts under controlled temperature conditions. Such methods form a further aspect of the invention.

The precise method of preparation may be varied taking into account factors such as the nature of the components and the form of the final product. Typically, the step of oxygenation will be carried out in respect of one or more liquid components of the formulation, although this may alternatively be carried out in respect of the final liquid formulation. As described above, it is also possible to oxygenate a thickened liquid (where this is flowable) using the oxygenation methodology as herein described.

As will be appreciated, the oxygenated formulations of the invention can therefore be produced according to different methods. Such methods include, but are not limited to, the following:
- production of the final liquid which is subjected to oxygenation immediately prior to packaging; and
- oxygenation of water or a physiological salt solution and addition of this to the remaining components of the liquid formulation prior to packaging.

Mixing of the various components of the formulations at low temperatures (e.g. in the range 2 to 25°C, preferably at about 4 to 5°C) and, preferably, under controlled pressure conditions is generally advisable to minimise the loss of oxygen. For example, mixing may be carried out in vials or containers with no head space and/or at elevated pressure (e.g. 5 bar). Stirring or agitation of the formulations during preparation should also be controlled, e.g. minimised, to avoid the loss of oxygen.

Where the formulations are thermogelling, low temperatures are generally required to facilitate dissolution of the thermogelling agent (i.e. the poloxamer or poloxamer blend) into the aqueous solution and to minimise any alterations to the nature of the poloxamer(s).

In one embodiment, solid poloxamer powder or granules may be dissolved in a cooled aqueous solution containing the dissolved oxygen (e.g. water or physiological saline) until a homogenous solution is obtained. At this point, the remaining components of the formulation may be added. Alternatively, these components may first be dissolved into the aqueous oxygenated solution which is subsequently mixed with the poloxamer (or poloxamer blend). A further method may involve the preparation of a highly concentrated poloxamer solution in which the poloxamer or poloxamer blend is first dissolved in a small volume of water prior to the addition of oxygenated water and other components. As described above, a yet further method may involve the production of the (non-oxygenated) liquid formulation by mixing of the various components, followed by oxygenation of this formulation (e.g. by passing this through an oxygenation apparatus as herein described).

For use *in vivo* the formulations herein described should be sterilised. This can be achieved by methods known in the art. The conditions for sterilisation should be selected such that the product maintains its thermogelling properties whilst minimising the level of viable microorganisms in the product during storage. For example, the separate components of the formulation may be sterilized prior to mixing. Sterilization of any solid poloxamer (e.g. powder or granules) may, for example, be achieved by electron beam radiation or gamma radiation. Alternatively, the final formulation may be sterilized once all components have been mixed and dissolved. In this case, sterilization may similarly be achieved by gamma or electron beam irradiation or by other means such as microfiltration using a filter having a small pore size (e.g. about 0.22 µm). The ability to filter the formulation will be dependent on its final viscosity, but when cooled sufficiently such that this is in a liquid state microfiltration will generally be feasible.

In another aspect the liquid formulations herein described may be incorporated into a suitable delivery device, for example these may be provided in, or as a component of, a dressing, bandage or any other suitable wound covering. In use, the delivery device may be applied to the target tissues (e.g. the surface of the skin) such that the liquid contained therein comes into contact with the underlying body tissues. Where the liquid is provided within the interior of the device this may flow to the exterior surface in order to provide body contact. On contact with the tissues, the flowable liquid forms a gel and releases dissolved oxygen.

In one embodiment, the liquid formulations may be soaked into an absorbent dressing, bandage or wound covering (e.g. a gauze), or a portion thereof, and packaged ready for use. The soaked dressing may be packaged under a vacuum or pressure.

Alternatively, the delivery device containing the formulation may be prepared at the point of use by application of the liquid formulation to a suitable wound covering (e.g. by soaking or immersion of the wound covering in the liquid formulation) immediately prior to application to the body tissues.

The liquid formulations herein described may be packaged in a suitable, sealed container or packaging which is sterilised, e.g. by steam sterilization (i.e. autoclaving) or gamma irradiation. Autoclaving may be carried out at a temperature in the range from 105 to 150°C, preferably 120 to 135°C for a period of time which is sufficient to kill microorganisms. Sterilization times are dependent on the type of item to be sterilized, e.g. metal, plastic, etc., but can be expected to be in the range of from 1 to 60 minutes, e.g. 4 to 45 minutes. Typical steam sterilizing temperatures may be 121°C or 132°C.

Suitable types of containers may be selected according to the nature of the formulation, and its intended use, e.g. the type of wound to be treated, the duration of treatment and whether multiple uses are envisaged. Suitable packaging includes vials, loaded syringes, tubes, pouches, bottles, etc. In each case these should be effectively sealed in order to avoid depletion of oxygen on storage. Vials may, for example, be provided with a suitable twist to break cap.

Packages may be intended for single or multiple use. Where these are intended for multiple use it is important that the remaining content of the package can be sealed after opening and following the delivery of each dose of formulation in order to maintain the sterility of the product and minimise the loss of oxygen. The use of an aerosol cannister, which includes a suitable propellant, may be suitable in this regard. Any known delivery systems in which any headspace is replaced with a vacuum or inert gas following the delivery of each dose may be used. Containers having a one-way pump are also suitable.

Alternatively, the formulations may be provided in individual doses, e.g. in sachets, small tubes or bottles which contain an amount sufficient for a single application to the skin. Single use ampoules are preferred.

Maintaining high and stable oxygen levels in the product when stored is essential. Suitable storage containers, lids and the materials used for their preparation should be chosen accordingly. These should have low susceptibility to penetration of gases, especially oxygen. Preferably these should be impermeable to gases. Suitable containers include glass jars, vials and tubes, and disposable plastic containers such as those made from polyethylene terephthalate (PET) or its copolymers. Optionally any plastic containers (e.g. those made from PET or its copolymers) may comprise additional components to enhance their gas barrier properties. Such materials are, for example, described in US 2007/0082156 (The Coca-Cola company) and WO 2010/068606 (The Coca-Cola company).

Ideally, any storage containers should have minimum oxygen permeability in order to maximise shelf life of the product. Typically a suitable shelf life is a minimum of about 6 months, preferably 6 to 12 months under ambient conditions. Shelf life may be extended by storage at lower temperatures, e.g. under refrigeration at a temperature in the range from 2 to 4°C. During storage for the intended shelf life, it is preferable that the oxygen content of the product should not be reduced by more than 25%.

As will be understood, since the formulations are intended to gel *in-situ,* it is important that these are stored at a temperature which is lower than the sol-gel transition temperature in order to keep these in the liquid state. The storage temperature may be determined having in mind the nature of the formulation and the gelling agent which is present.

The formulations herein described may be applied to any compromised tissues where delivery of oxygen is desirable. The method of delivery will be dependent on the form of the product, i.e. whether this is a liquid or provided as a component in a delivery vehicle as herein described. For any therapeutic use, in order to maintain the sterility of the product it is generally envisaged that these should be applied by sterile means. For example, where these are supplied as a liquid, these may be applied to the target area using an applicator (e.g. from a syringe), or by methods such as spraying.

Compromised tissues include those that have an interrupted supply of blood and thus an inadequate supply of oxygen. Wounds are one example of compromised tissues and typically involve interruption in the integrity of the skin. When skin is damaged or removed, e.g. removed by surgery, burned, lacerated or abraided, its protective function is lost. All types of skin wound may be treated in accordance with the invention, including both acute and chronic wounds.

Acute wounds are usually tissue injuries that heal completely with minimal scarring within the expected timeframe, e.g. up to 10 days. Primary causes of acute wounds include mechanical injuries due to external factors such as abrasions and tears which are caused by frictional contact between the skin and hard surfaces. Mechanical injuries also include penetrating wounds caused by knives and surgical wounds caused by surgical incision (e.g. in the removal of tumors). Acute wounds also include burns and chemical injuries, such as those which may arise from radiation, electricity, corrosive chemicals and thermal sources (both hot and cold). Burn wounds may be classified according to their severity, e.g. as first, second or third degree burns.

Chronic wounds arise from tissue injuries that heal slowly, e.g. injuries that have not healed after about 12 weeks, and often recur. Such wounds typically fail to heal due to repeated tissue injury or underlying physiological conditions such as diabetes, obesity, malignancies, persistent infections, poor primary treatment and other patient-related factors. Chronic wounds include skin ulcers, such as decubitis ulcers (e.g. bedsores or pressure sores), leg ulcers (whether venous, arterial, ischaemic or traumatic in origin), and diabetic ulcers. Venous leg ulcers are caused by venous insufficiency due to malfunctioning of the valves in the veins in the leg and may lead to pulmonary embolia which is a life-threatening condition. They are costly to treat, often requiring hospitalization. Arterial leg ulcers are caused by poor functioning or occlusion of the arteries in the leg and may arise from conditions such as arteriosclerosis. Diabetic ulcers arise from impaired microcirculation as a result of diabetes. In the case of diabetic ulcers, failure to heal can often lead to loss of a limb.

Wounds may also be classified according to the number of skin layers and area of skin which is affected. In a superficial wound the injury affects the epidermal skin surface alone. Injury involving both the epidermis and the deeper dermal layers, including the blood vessels, sweat glands and hair follicles, may be referred to as a partial thickness wound. A full thickness wound occurs when the underlying subcutaneous fat or deeper tissues are damaged in addition to the epidermis and dermal layers.

When used in the treatment of wounds, the formulations of the invention increase the rate of wound healing through improved oxygenation, whilst simultaneously retaining moisture at the wound site and protecting against infection.

Use of the formulations and delivery devices disclosed herein further extends to the treatment of other conditions in which body tissues are damaged or otherwise compromised and need to regenerate, and any conditions which tend to the decrease the normal tissue oxygen levels. Other conditions which may be treated include skin disorders (e.g. psoriasis, acne, rosacea, and other dermatological conditions such as atopic dermatitis), skin sores, and tissue necrosis.

Acne is one of the most common skin disorders. In its milder forms it is a superficial disorder which is accompanied by spotty skin irritations. However, more severe acne involves bacterial invasion of the pilosebaceous follicles which results in the formation of inflammatory lesions such as papules, pustules and infected cysts. Both inflammatory and non-inflammatory acne may be treated according to the invention. Representative types of acne which may be treated using the formulations herein described include acne vulgaris, acne rosacea, acne conglobate, acne papulosa and premenstrual acne, in particular acne vulgaris which is associated with inflammation of the pilosebaceous follicles. Acne may occur on the back, chest, upper arms and/or face and the formulations herein described may be used for treating any of these areas of the body, especially the face and back.

The formulations according to the invention also find use in cosmetic methods, for example in methods of improving or otherwise enhancing the appearance of the skin. Cosmetic methods include improving or otherwise enhancing the appearance of the skin. For example, the formulations may be used for their anti-aging activity, anti-wrinkle activity, skin-softening effects, or their ability to reduce hyper-pigmentation of the skin. Aging of the skin may result not only from internal factors but also external factors such as environmental conditions (e.g. exposure of the skin to sun, wind, humidity, etc.). Aging results in skin changes which include roughness, dryness, hyper-pigmentation, fine lines and wrinkles.

As will be understood, certain milder forms of acne (e.g. blackheads and/or white heads) may not always be considered to be a disease and so treatment of these may be carried out purely for cosmetic reasons. The cosmetic treatment of acne, for example where acne is relatively infrequent and/or not widespread (i.e. only a few spots occur), is encompassed by the invention. Such cosmetic methods will typically be targeted to the treatment of the face.

Compromised tissues can also result from other disturbances in the normal functioning of the skin. For example, damage can arise from an internal metabolic dysfunction such as diabetes, an inflammatory response, or a circulatory disorder, or from an external irritant, such as a chemical irritant, UV damage, etc.

Inflammatory skin diseases are a common problem in dermatology and these may be treated using the formulations herein described. These diseases may take a number of different forms, from occasional rashes accompanied by skin itching and redness, to chronic conditions such as dermatitis (eczema), rosacea, seborrheic dermatitis, and psoriasis. Skin inflammation can be characterised as acute or chronic. Acute inflammation can result from exposure to UV radiation, ionising radiation, allergens, or from contact with chemical irritants (e.g. soaps, hair dyes, etc.). This type of inflammation is typically resolved within 1 to 2 weeks with little accompanying tissue destruction. In contrast, chronic inflammation results from a sustained immune cell mediated inflammatory response within the skin itself. This inflammation is long lasting and can cause significant and serious tissue destruction. Infection is a complication of many inflammatory skin disorders in which the protective layers of the skin are broken, e.g. in conditions such as eczema.

Use of the formulations also extends to the treatment of both aerobic and anaerobic bacterial and fungal infections of the skin due to the toxicity of oxygen to such pathogenic organisms. Examples of conditions associated with bacterial infections include cellulitis, infections in chronic wounds, gas gangrene, necrotizing fasciitis (infection by enterococcus, enterobacteriacea, clostridium, B. fragilis, streptococcus, pyogenis). Examples of invasive fungal infections include those associated with mucorales, aspergilus.

In one embodiment, the formulations and methods herein described may thus be used to treat an infection (e.g. an anaerobic or aerobic infection). This will involve application of the formulation to an area of infected skin, e.g. a skin lesion which harbors bacteria. Infected tissues can often become inflamed. By treating infected tissues, inflammation can be prevented or at least reduced.

Examples of conditions in which anaerobic bacteria may be found include gangrene and ulcers. Anaerobic bacteria may also be found in infected wounds and areas of skin burn. Wounds and burns are particularly susceptible to infection where the tissue is destroyed or badly damaged, such as in second or third degree burns. In such cases, application of the formulations of the invention can be used to prevent bacterial infection as well as act therapeutically to heal the damaged tissue.

Anaerobic bacterial infections which may be treated and/or prevented using the formulations of the invention include Pseudomonas species, Bacteroides species, and Clostridium species, Enterococcus species, Enterobacteriacea species, Bacillus species, Streptococcus species, etc.

In one embodiment, the formulations and methods herein described may be used to prevent the formation of a bacterial biofilm and/or to treat a bacterial biofilm on a body surface. Treatment will typically involve disruption, removal or detachment of at least part of the biofilm from the body surface.

The subject to be treated may be any mammal. Although typically the subject will be a human, the methods herein described are equally suited to the treatment of non-human mammals. Veterinary use of the formulations is thus envisaged within the scope of the invention.

The formulations may be applied in a variety of different ways depending on factors such as the area to be treated, the nature of the condition, the subject to be treated, etc. These may be applied to any area of the body including the face, chest, arms, legs or hands. Typically, they will be applied to the skin. For use as a cosmetic, it is envisaged that this would primarily be applied to the face. The method of application to the skin may be by spraying, rubbing, soaking, immersion, continuous perfusion, injection, etc. Dependent on the viscosity of the formulation, for cosmetic use the formulation will normally be applied by the hands or fingers.

For any therapeutic use, the formulations may be applied by the fingers, however, in order to maintain sterility it is generally envisaged that these would be applied by sterile means, for example using an applicator. Applicators known for use in applying dermal products may be used depending on the nature of the formulation, especially its viscosity. For example, this may be applied with a spatula (e.g. where this is a thickened or highly viscous liquid) or possibly sprayed or dripped onto the surface of the skin.

In cases where the composition is intended for use in treating a wound, following application of the composition directly to the wound this would typically be covered by a dressing.

The formulations may be applied in the form of a liquid which *in-situ* forms a gel which contacts the target tissue (e.g. a wound) and serves to form a "primary dressing". Typically this will require a secondary dressing to protect the gel and to ensure that this remains in place for the duration of the treatment. The secondary dressing should be flexible and able to conform to the wound site. Typically this dressing will take the form of a sheet of conventional wound dressing material which may be cut to the appropriate size and shape depending on the area of compromised tissue to be treated.

Any secondary dressing should ideally be of limited permeability to water and/or oxygen, e.g. this should be substantially impermeable to water and/or oxygen. The use of an occlusive dressing not only ensures that the dissolved oxygen present in the underlying gel is delivered to the skin, but it also serves to maintain a moist healing environment for the wound. By "substantially impermeable to oxygen" is meant that less than 25% of the oxygen content of the gel may be lost through the dressing.

In dealing with repair and healing of compromised tissues, e.g. wounds, it may be necessary to control exudate from the wound. This may involve drainage of exudate from the wound or absorption using a suitably absorbent dressing. Maintaining an optimal level of moisture at the site of the compromised tissue is also important, particularly in cases where there is heavy production of exudate. The use of a dressing helps to achieve this. In one embodiment the secondary dressing may thus be highly absorbent, particularly in the case of treating any wound with a high level of exudate. Where it is highly absorbent, this will typically be applied to the wound once the formulation has formed a gel at the wound site in order to minimise the uptake of the liquid formulation into the dressing and so maximise its contact with the tissues.

Examples of suitable dressings are known in the art and may readily be selected according to the type of wound, its size and location. Known dressings include both synthetic and biological dressings, such as synthetic films, alginates, hydrocolloids, hydrogels and collagen dressings. Those that are substantially impermeable to the passage of oxygen include polyesters and polyolefins.

If desired the wound may also be covered with a compression bandage. This may be beneficial, for example, when treating venous ulcers.

In use, the formulation is applied directly to the wound site or as close as possible to this. Preferably this should be in direct contact with the wound bed. A suitable secondary dressing is then applied over the formulation and, if required, secured in place using tape, gauze or any other suitable means to secure this to intact skin. The secondary dressing may be temporary so that this may, if required, be removed and replaced with a fresh dressing. In one embodiment, the secondary dressing may be coated, in part, with an adhesive which is capable of securing this to the skin. For example, the dressing may have adhesive around its periphery. Suitable adhesive materials are known in the art and include, for example, polyisobutylene, polysilicone and polyacrylate. Where the dressing is supplied with an adhesive portion, this will generally also have a release liner, e.g. a siliconised polyester film, which is removed prior to use.

The duration of treatment will depend on the nature of the wound and the oxygen content of the formulation applied to the skin. Typically, the dressing may be used on the wound for several days, e.g. up to 3 days. Use of the dressing for several days further reduces the cost of the treatment and reduces the trauma involved in changing of the dressing (e.g. where this may be required to be changed every day or several times a day). Delivery of oxygen from the dressing is controlled. Controlled release relates to a release of oxygen over a predetermined period of time from 7 hours to 2 days. The delivery of oxygen is preferably substantially continuous during this period meaning the delivery is substantially uninterrupted.

In some cases, a further application of the formulation may be desirable and this can be repeated as often as required. In order to change the dressing, the oxygen-depleted gel may easily be removed from the wound by gentle irrigation with a physiologically acceptable solution, such as sterile water or saline solution. Oxygenated water or oxygenated saline may also be used for this purpose. Irrigation of the wound between changing of the dressing also serves to cleanse the wound to remove dead or necrotic tissue.

Wound healing has several different phases which may not all be targeted by a particular formulation or dressing. Accordingly, the nature of the formulation and any secondary dressing may be adjusted not only for different types of wound (e.g. acute, chronic, dry, exuding, etc.) but also for different stages in the healing of the wound. This includes, in particular, varying the oxygen content of the different formulations for the different stages of treatment. During the early stages of wound healing, low pO₂ (hypoxia) is an essential stimulator of growth factors, cytokines, gene activation and angiogenesis, whereas normal (normoxia) or increased (hyperoxia) levels of pO₂ are more favorable during the subsequent stages of wound healing. Fibroblast and endothelial cell proliferation, for instance, occurs best at a pO₂ of 30 to 80 mm Hg and collagen synthesis, neovascularization and epithelialization all require a pO₂ between 20 and 60 mm Hg.

Due to their ease of use, the wound treatments herein described may be used as home care, thereby reducing treatment costs and avoiding the need for hospitalization of patients. These also allow for full mobility for patients during treatment without the need for hospitalization, oxygen tanks or additional equipment. This increases the quality of life for patients.

The formulations herein described and the resulting gels may also find use in *in vitro* methods of cell and tissue culturing of cells or tissues which demand an oxygenated cell medium. Transformation of the liquid into a gel (i.e. at elevated temperatures suitable for use in tissue or cell culturing) provides a suitable support or scaffold for the cells or tissues. When used in this way, it is preferable that the *in-situ* gelling agent is thermoreversibly gelling. Flushing of the gel with saline or any other suitable rinsing solution at a reduced temperature (e.g. ambient temperature) causes the gel to solubilise thus enabling separation of the cells or tissues, for example prior to implantation into the body.

Also described herein is an *in vitro* method of cell or tissue culturing, said method comprising the step of contacting said cells or tissues with an oxygenated gel obtained (or obtainable) from a liquid formulation as herein described.

The formulations herein described are also intended for dermal use on the skin of a mammal, preferably a human subject. As such, these are compatible not only with the skin, but also with mucous membranes, nails and hair. Typically, these will also be non-irritant and well-tolerated when applied to the skin. When used for cosmetic purposes, the formulations are applied to the subject's skin, which may include the face, neck, chest, arms, legs or hands. Primarily, these may be applied to the face, for example by the hands and fingers and gently spread onto the target area. These may be left in place for a suitable period of time in order to allow for *in-situ* gelling and delivery of the dissolved oxygen to the skin prior to removal, e.g. by washing the skin with water.

The invention will now be described further with reference to the following nonlimiting Examples and the accompanying figures in which:

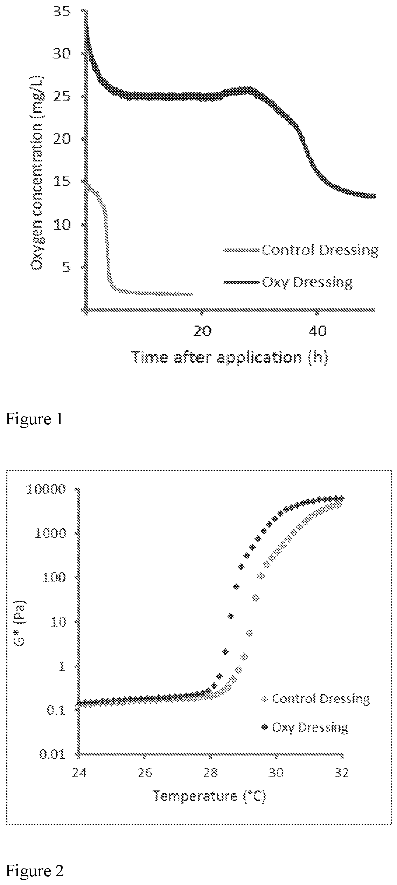

Figure 1 shows the stability of a formulation in accordance with the invention ("Oxy Dressing") when applied on a skin model. Dissolved oxygen levels in the "Oxy Dressing" with an original dissolved oxygen level of 32 mg/L were kept above 25 mg/L for more than 30 hours at 35°C when kept in a closed system. The control dressing had an original dissolved oxygen concentration of 14 mg/L, and dropped to 2.5 mg/L after approximately 5 hours.
Figure 2 shows the rheology of a formulation in accordance with the invention ("Oxy Dressing"). The influence of viscosity (G*: shear modulus complex component) on increased temperature for Lutrol F127 in MilliQ and in "Oxy Water" is shown. The presented data are representative measurements of one individual sample for each formulation.

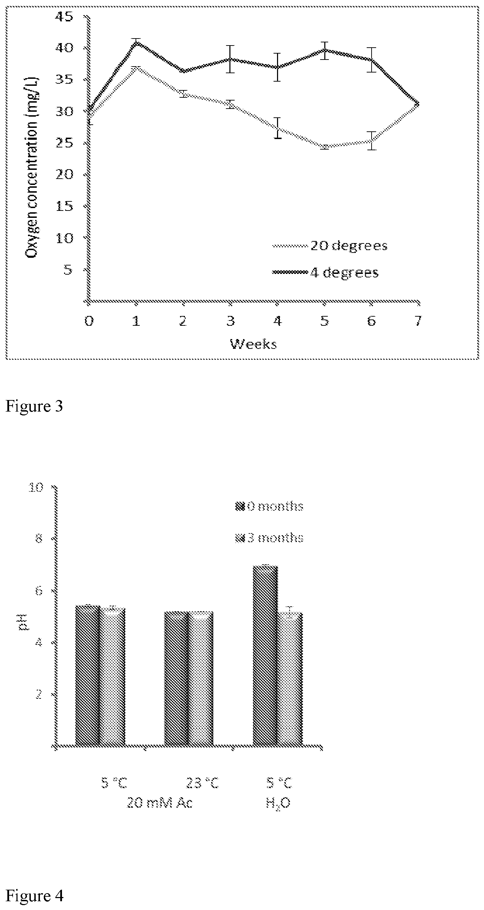

Figure 3 shows the shelf life of a formulation in accordance with the invention ("Oxy Dressing"). Dissolved oxygen was measured by Winkler titration in the "Oxy Dressing" after storage at room temperature (20°C) or in the fridge (4°C) for up to 7 weeks. The "Oxy Dressing" maintained stable dissolved oxygen levels above 30 mg/L when stored at 4°C, and above 20 mg/L when stored at room temperature in capped glass vials for 8 weeks. Data are presented as average ± SD.
Figure 4 shows the pH stability of a formulation in accordance with the invention ("Oxy Dressing"). The pH value of Lutrol F127 formulated in 20 mM acetate buffer and MilliQ water, stored at 5°C and 23°C for 3 months, is shown. The "Oxy Dressing" kept a stable pH for 3 months when prepared in 20mM acetate buffer. Results are presented as averages and standard deviations from individual measurements of 3-9 different samples.

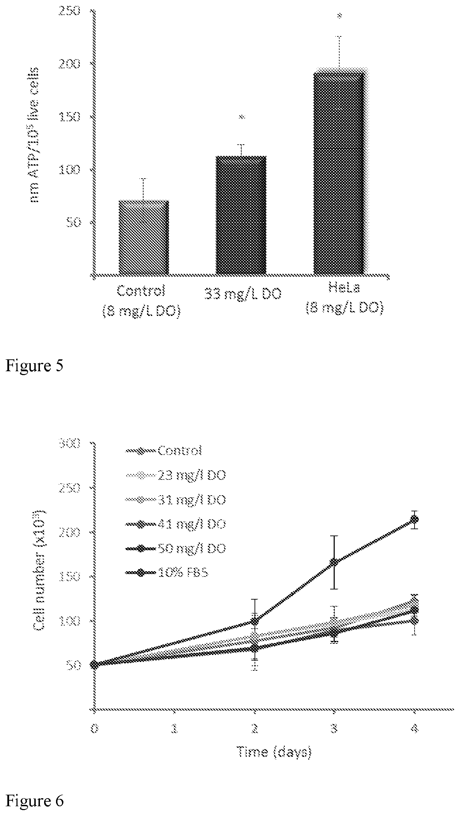

Figure 5 shows the *in vitro* effect of dissolved oxygen (DO) on human skin fibroblasts - Adenosine triphosphate (ATP). ATP levels, presented as average nmol/10⁵ live cells ± SD, were measured in human skin fibroblasts incubated for 4 hours in control medium (DMEM w/10% FBS, 8 mg/L DO), DMEM w/10% FBS and 33.0 mg/L DO, or positive control (HeLa cells, DMEM w/10% FBS, 8 mg/L DO). The cells were re-stimulated with the respective conditions at 1, 2 and 3 hours. The cells treated with 33 mg/L DO (113.1 nm ATP/10⁵ cells) and positive control (191.6 nm ATP/10⁵ cells) had significantly higher ATP levels than the control (70.9 nm/10⁵ cells) after 4 h (n=3, *p<0.05).
Figure 6 shows the *in vitro* effect of dissolved oxygen (DO) on human skin fibroblasts - Proliferation. Cells were grown in DMEM w/1% FBS with (23-50 mg/L) or without (11 mg/L) high levels of DO, or in DMEM w/10% FBS (11 mg/L, positive control). Medium was changed every day. At Day 2, 3 and 4 cells were harvested and manually counted using a hemocytometer. Treating cells with DO up to 50 mg/L did not significantly alter the proliferation rate of human skin fibroblasts when compared to control. Data are expressed as average ± SD (n=4).

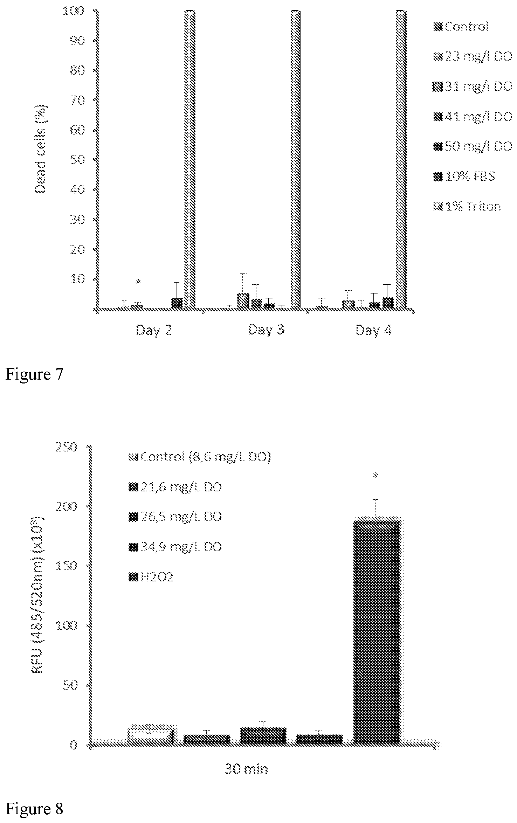

Figure 7 shows the *in vitro* effect of dissolved oxygen (DO) on human skin fibroblasts - Cell viability. Cells were grown in DMEM w/1% FBS with (23-50 mg/L) or without (11 mg/L) high levels of DO, or in DMEM w/10% FBS. 1% Triton X was used as positive control. Medium was changed every day. At Day 2, 3 and 4 cells were harvested, mixed with trypan blue and dead cells were counted using a TC20 Automated cell counter. Treating cells with DO up to 50 mg/L did not significantly alter the number of dead cells compared to control over the course of 4 days. Data are expressed as the percentage of dead cells relative to total cells and represent the average ± SD (n=4, *p<0.05).
Figure 8 shows the *in vitro* effect of dissolved oxygen (DO) on human skin fibroblasts - Reactive Oxygen Species (ROS). ROS levels, presented as relative fluorescence (×10³) ± SD, were measured in human skin fibroblasts incubated 30 min in control (DMEM w/5% FBS, 8.6 mg/L DO), 3 different concentrations of DO (DMEM w/5% FBS, 21.6, 26.5, or 34.9 mg/L DO) or H₂O₂, positive control (DMEM w/5%FBS, 8.6 mg/L DO and 500µM H₂O₂). The positive control (H₂O₂) had significantly higher levels of ROS compared to control. There were no significant differences between the ROS production in the cells treated with DO (21-35 mg/L) and control (n=3, *p<0.05).

### Examples

### Example 1 - Preparation of oxygenated water ('Oxywater')

### Method:

1. Reverse osmosis (RO) water was chilled to 2-4°C.
2. Chilled water was fed into an oxygenation device as described in WO 2016/071691.
3. Oxygen gas was at the same time fed into the mixing chamber of the device.
4. Water and O₂ gas were passed through the venturi and O₂ gas dissolved in the water.
5. The gas input may vary depending on the flow rate of the gas and the process pressure and this may be used to adjust the O₂ content of the water. For this production the process pressure employed was 42 psi.
6. The water was produced by continuous circulation through the device until a dissolved oxygen content of 100 mg/L was reached.
7. The oxygenated water was bottled in glass bottles and stored at ambient temperature.

Oxygen content of the water was varied by adjusting the flow rate and pressure of O₂ gas. In this way it was possible to prepare oxygenated water having a range of O₂ contents according to need.

### Example 2 - Preparation of thermogelling formulations and determination of O₂ levels

### Materials:

Oxygenated water (approx. 70 mg/L oxygen content) - prepared by a method analogous to Example 1
Poloxamer - Lutrol F127^{®} (BASF)
TRIS buffer (Merck)
5M HCl

### Method:

During preparation of the formulations the oxygenated water was handled with care in order to retain the high oxygen content. A fresh bottle of oxygenated water was opened for each formulation. Prior to use, the bottles were refrigerated at 2-4°C in order to chill the water before preparation of the formulations.

A concentrated solution of TRIS buffer was prepared by adding 8-9 ml oxygenated water to 12.1 g TRIS base. The pH was adjusted to 7.5 using 5M HCl and the solution was made up to a volume of 10 ml by adding additional oxygenated water. In a mixing vial, 40 ml of a concentrated (40 wt.%) poloxamer solution was prepared by dissolving the poloxamer powder in a solution containing the 10 ml of TRIS buffer solution and 30 ml of oxygenated water.

The final formulation was prepared by dilution of the concentrated poloxamer solution (40 ml) to the final concentration (16 wt.% poloxamer) with fresh, ice-cooled oxygenated water (60 ml).

All steps were carried out without agitation. In order to minimise the tendency for bubble formation and loss of oxygen all work was carried out at 5°C. Head space in the mixing vial was kept to a minimum. In order to further minimise the loss of oxygen, mixing may be done under pressure (air or oxygen).

### Analysis of oxygen content:

Prior to use, the oxygen content of the water was measured by Winkler titration and using an oxygen meter available from Orion, Thermo Scientific. For measurements using the oxygen meter, the oxygenated water was mixed 50:50 with MilliQ water and oxygenation levels were measured as 33-41 mg/L, i.e. 66-82 mg/L after compensation for the dilution.

Oxygen content of the formulations during gelling was monitored using an Oxygen meter developed by SP Technical Research Institute of Sweden. The oxygen meter (Oxymeter) supplied by Orion, Thermo Scientific was used to measure the oxygen content of the formulation before applying this to the skin model.

### Gelling with temperature.

Thermogelling was achieved either by lowering the vial containing the liquid formulation into a beaker of heated water at 35-37°C, or by direct application of the formulation onto the skin.

### Results:

Thermogelling formulations having final oxygen contents in the range of from 25 to 30 mg/L were prepared according to the protocol described above. The resulting gels were capable of retaining the oxygen content at about 20 mg/L for at least 2 hours after application (i.e. following gel formation). When the gel was prevented from drying (i.e. stored in a closed system), oxygen levels above 25 mg/L were retained for at least 30 hours.

### Conclusions:

The formulation protocol provided a thermogelling formulation of Oxywater with a final oxygen content of 25-30 mg/L with the potential to be used in wound healing and cosmetic applications.

The thermogelling formulation could oxygenate tissues for at least 30 hours under certain conditions - i.e. if the gel does not dry out and provided the oxygen is prevented from escaping to the surrounding air.

### Example 3 - Preparation of thermogelling formulations and determination of O₂ levels

### Materials:

Oxygenated water (approx. 70 mg/L oxygen content) - prepared by a method analogous to Example 1
Poloxamer - Lutrol F127^{®} (BASF)
Acetic acid (Sigma-Aldrich)
Sodium acetate
10N NaOH

### Method:

A concentrated solution of acetate buffer was prepared by adding oxygenated water to 54.43 g sodium acetate and 12 ml acetic acid to produce a total volume of 180 ml. The pH was adjusted to 7.5 using 10N NaOH and the solution was made up to a volume of 200 ml by adding additional oxygenated water.

In a mixing vial, 40 ml of a concentrated (40 wt.%) poloxamer solution was prepared by dissolving the poloxamer powder in a solution containing 10 ml of the acetate buffer solution and 30 ml of oxygenated water.

The final formulation was prepared by dilution of the concentrated poloxamer solution (40 ml) to the final concentration (16 wt.% poloxamer) with fresh, ice-cooled oxygenated water (60 ml).

### Example 4 - Preparation of thermogelling formulations

Other poloxamer-based formulations were prepared with oxygenated water containing 58 mg/L dissolved O₂. In a mixing vial, 40 ml of a concentrated (40 wt.%) poloxamer solution was prepared by dissolving the poloxamer powder in a solution containing 40 ml of the oxygenated water. The final formulations were prepared by dilution of the concentrated poloxamer solution (40 ml) to the final concentration with fresh, ice-cooled oxygenated water.

Thermogelling was assessed either by lowering a vial containing the formulation into a water bath at 35-37°C, by direct application of the formulation onto the skin, or with a kinexus Pro Rheometer with a plate-plate 20 nm diameter.

Table 1 provides details of the formulations and their thermogelling properties. Formulations having an O2 conc. of less than 20 mg/L do not form part of the invention.

**Table 1:**

| **Total Poloxamer (wt.%)** | **F127 (wt.%)** | **F68 (wt.%)** | **O2 conc. (mg/L)¹** | **Tgel (WB)²** | **Tgel (Rheo)³** | **G" (Pa) at 35°C** | **G' (Pa) at 35°C** | **Comment-application to skin⁴** |
|---|---|---|---|---|---|---|---|---|
| 16 | 16 | 0 | 23 | 32 | 31 | 805 | 9158 | Low viscosity, slightly "runny" after application |
| 20 | 18 | 2 | 20 | 36 | - | - | - | Low viscosity, very "runny" after application |
| 20 | 18.5 | 1.5 | 18 | 32 | 28 | 861 | 17140 | Low viscosity, slightly "runny" after application |
| 20 | 19 | 1 | 19 | 28 | 25 | 865 | 16930 | Low viscosity, slightly "runny" after application |
| 25 | 20 | 5 | 13 | 37 | 31 | 1167 | 13360 | Low viscosity, "runny" after application |

| | | | | | | | | |
|---|---|---|---|---|---|---|---|---|
| ¹O₂ levels for the formulations prior to gelling ²Gelling temperature in water bath ³Tgel measured by rheology (Tgel (Rheo)) and in the water bath (Tgel (WE)) differ due to differences in the Tgel definition ⁴ consistency immediately after application of the formulation to the skin | | | | | | | | |

### Conclusions:

- In all cases, the low viscosity on application to the skin was acceptable.
- Increasing the poloxamer concentration from 16 wt.% to 20 wt.% leads to a higher gel strength and faster gelling onset. Further increasing the poloxamer concentration from 20 wt.% to 25 wt.% does not change the gel strength significantly.
- The oxygen content in the gels can be increased if the oxygenated water for use in the formulation has a higher oxygen content.
- The presence of oxygen does not significantly influence the gelling properties.
- When evaluating the gels on the skin, only very small differences were detected between the formulations.

### Example 5 - Preparation of thermogelling formulation and testing

### Methods:

### Production of oxygenated water ("Oxy Water ")

Water with elevated levels of dissolved oxygen was produced as described in WO 2016/07169. Reverse Osmosis (RO) water and oxygen was fed into a chamber of an oxygenation device. The water and oxygen were mixed and subsequently passed through a piping system including a venturi. The system ran continuously at 2.9 bar and was fed with 98% O₂. At the outlet, oxygenated water containing up to 100 mg/L dissolved oxygen was collected, bottled in glass bottles and stored at room temperature until use.

### Winkler titration for determination of dissolved oxygen concentrations

Samples of the Oxy Water were mixed with manganese sulphate (2.2 M) and alkaline iodide azide (12 M NaOH, 0.86 M KI). The precipitate was allowed to settle halfway two times before 98% sulphuric acid was added, giving a dark amber colour. The solution was then titrated with sodium thiosulfate (0.0375 M). Starch indicator solution was added when the solution reached a light yellow colour. Each 1.0 ml of sodium thiosulfate used was equivalent to 3 mg/L dissolved oxygen.

### Formulation of oxygenated in-situ gelling dressing ("Oxy Dressing")

The Oxy Dressing was formulated with Oxy Water (~65 mg/L) and Lutrol F127 (BASF). A fresh bottle of oxygenated water at 2-4°C was opened for each formulation. In a mixing vial, concentrated (40-50 wt.%) Lutrol F 127 solution was prepared by dissolving the poloxamer powder in the oxygenated water under gentle agitation. When the poloxamer was dissolved, the stock solution was diluted with Oxy Water (both 0°C) to a final concentration of 15-16 wt%.

The Oxy Dressing was also formulated in 20 mM acetate buffer for pH control. 30 wt.% of Lutrol F127 was dissolved in a 38 mM acetate buffer by gentle mixing in an ice bath (0°C) to minimise the viscosity of the blend. After dissolution of the Lutrol F127 in the stock solution, the 30 wt.% polymer solution was mixed with Oxy Water cooled to 0°C to a final concentration of 15-16 wt% Lutrol F127.

### Oxygen stability after application

An oxygen optode developed by RISE (Borås, Sweden) was used to evaluate the oxygen concentration and stability of the Oxy Dressing over time when applied at a temperature controlled surface of 35°C (skin surface temperature). The optode was assembled on to a tip of a fibre optic probe (NeoFox, Ocean Optics) and mounted inverted (facing upwards) on a heating block controlling the surface temperature. The principle for the measurements was based on phase fluometry. Thus, the dye in the optode film was excited with a pulsed blue LED that caused a luminescence delay (phase shift) relative to the pulsed LED frequency, which is dependent on the oxygen concentration. A high oxygen level reduces, and low oxygen levels prolongs, the luminescent lifetimes as well as phase shifts. Oxy Water and calibrating solutions were measured using a brass pipe (15ml) assembled onto a seal surrounding the fibre optic tip. Calibration of the heated (35°C) optode was made by exposing the assembled sensor to argon and oxygen saturated ultra-purified water, respectively at 35°C. The oxygen contents of the calibrating solutions were also confirmed with Winkler titration. During measurements of the Oxy Dressing, a smaller black seal was used as a container during gel application facilitating an exact volume (1.5 ml) and surface area (3.5 cm²). The area of the gel measured corresponded to an uncured thickness of 4 mm. To avoid the gel from drying out, measurements were also performed when covering the gel with a glass lid. Temperature and oxygen levels were simultaneously and continuously measured during the experiments that lasted between 2-50 hours. Humidity and temperature in the room were determined before and after the measurements (29.7 % relative humidity and 21.5°C). Formulations prepared from MilliQ H₂O served as a control.

### Shelf life testing

For shelf life stability testing, the Oxy Dressing was gently filled in capped glass vials (Agilent HS, crimp, RB 20 ml with Hdspc Al crmp cap, PTFE/Si) with minimum head spacing, and stored at 20°C or at 4°C for up to 7 weeks. The dissolved oxygen concentrations were measured weekly by Winkler titration. Formulations prepared from MilliQ H₂O served as a control.

### Gelling properties

Thermogelling was assessed either by visual inspection, by lowering a vial containing the formulation into a water bath at 35-37°C, by direct application of the formulation onto the skin, or with a kinexus Pro Rheometer with a cone-plate (4 degrees, 40 mm diameter). When using the kinexus Pro Rheometer, 1.9 ml of formulation was applied for each measurement and the temperature was increased 1 degree /min at a frequency of 0.3 Hz and a deformation of 1 Pa.

### pH stability testing

The Oxy Dressing formulated in 20 mM acetate buffer was stored in capped glass vials at room temperature (23°C) and at 5°C for up to 3 months and pH was assessed regularly and compared to the pH of a 16 wt.% Lutrol F 127 solution in MilliQ water.

### Statistics

Winkler analysis was performed on 5 samples for each condition. Oxygen stability after application and rheology studies were performed on individual samples. The shelf life study was performed on two samples per group per condition and presented as average ± standard deviation. pH stability is presented as average and standard deviations from individual measurements of 3-9 different samples. Results were analysed statistically in SPSS using two-way analysis with unpaired t-test or ANOVA with Bonferroni post hoc test comparing the average values of each experiment. Values of p < 0.05 were considered statistically significant.

### Results:

The dissolved oxygen levels in the Oxy Dressing remained stable above 25 mg/L dissolved oxygen for more than 30 hours when applied on a skin model system with controlled temperature at 35°C and covered with a glass lid (see Figure 1). The covered Oxy Dressing did not dry out after 170 hours of operation, ending with an oxygen holding capacity exceeding 13 mg/L. The rheology and gelling properties of the dressing were not significantly influenced by the dissolved oxygen as shown in Figure 2.

The Oxy Dressing maintained stable dissolved oxygen levels above 30 mg/L when stored at 4°C, and above 20 mg/L when stored at room temperature (20°C) in capped glass vials for 7 weeks (see Figure 3).

The results in Figure 4 show that is possible to control the pH of the Oxy Dressing by formulating in a buffer and that the pH in the formulation is stable over time when stored at room temperature and under refrigerated conditions.

### Conclusions:

In the present study a highly oxygenated and pH controlled, thermosensitive topical dressing (Oxy Dressing) was formulated and tested. The Oxy Dressing has high and stable levels of oxygen for more than 30 hours and can efficiently oxygenate tissue for the same period of time. The shelf life studies show that the thermosensitive *in situ* forming dressing can retain the high dissolved oxygen content when stored.

Preparation of the Oxy Dressing in a buffer enables pH control, with stable pH over time. While normal skin has a pH of approximately 5.5, chronic wounds have been shown to have an alkaline pH. An acidic environment in the wound has been shown to control wound infection, increase antimicrobial activity, altering protease activity, enhancing fibroblast growth *in vitro,* releasing oxygen, reducing toxicity of bacterial end products, and enhancing epithelization and angiogenesis (Percival et al., Wound Repair Regen. 22(2): 174-86, 2014). In light of the importance of pH to wound healing, lowering the pH can itself be favourable for healing of wounds, and give an additional advantage to the wound healing properties of the Oxy Dressing.

### Example 6 - Testing of oxygenated water

The effects of oxygen on wound healing are well established. Nevertheless, the understanding of the mechanisms is still limited and requires further evaluation, thus *in vitro* studies of the effect of dissolved oxygen on human skin cells was performed.

### Methods:

### Cultivation, cells

Human skin fibroblasts (HSF) and HeLA cells (ATCC) were grown in complete cell medium (DMEM) with 10% Fetal bovine serum (FBS) and 1% penicillin/streptomycin (PenStrep) (Sigma Aldrich). At confluence, cells were detached using 5% trypsin -1mM ethylene diamine tetraacetic acid (EDTA) (Sigma Aldrich) and reseeded. Cells were cultured at 37°C in an incubator with humidified atmosphere and 5% CO₂.

### Preparation of oxygenated cell medium

Oxygenated medium was prepared using powder cell medium, diluted with Oxy Water prepared according to Example 5. The mixing ratio depended on the desired concentration of dissolved oxygen in the final cell medium. DMEM or DMEM medium without phenol red was used (Sigma Aldrich). The medium was supplied with additives and FBS. The phenol red free cell medium was used for the ROS experiment to avoid disturbance of the fluorescent readings.

### ATP concentration

HSF and HeLa cells (positive control) were seeded at a concentration of 2.5×10⁵ cells/9.5cm² and grown to confluence for 48 hours. The experiment included wells for ATP quantification and corresponding wells for cell counting. The cells were treated with complete cell medium (10% FBS) with dissolved oxygen: 8mg/L or 33 mg/L. After stimulation, the cells were left at room temperature for 30 min, followed by incubation at 37°C. The cells were re-stimulated with the respective treatments after 1, 2 and 3 hours. After 4 hours, the cells were rinsed with PBS and lysed on ice with lysis buffer (200 mM Tris, pH 7.5, 2 M NaCl, 20 mM EDTA, 0.2% Triton X-100). The lysate was centrifuged at 10 000 x g for 5 min. The luminescence was read at a plate reader (FLUOStar Omega, BMG Labtech) and compared to an ATP standard curve (ATP Determination Kit, ThermoFisher, USA). Cells in corresponding wells were harvested, trypan blue (Sigma Aldrich) added and subsequently the number of cells was determined by manually counting live and dead cells using a hemocytometer. The data were corrected for blank readings and presented as nm ATP/10⁵ live cells.

### Proliferation and cell viability

HSFs were seeded at a density of 5×10⁴ cells/25cm² and left overnight. The cells were treated with control cell medium (1% FBS, dissolved oxygen: 11 mg/L), oxygenated cell medium (1% FBS, dissolved oxygen: 23, 31, 41, 50 mg/L), or 10% FBS, dissolved oxygen: 10 mg/L. After stimulation, the cells were left at room temperature for 30 min, followed by incubation at 37°C. The treatment was repeated every 24 hours up to 4 days. On day 2, 3 and 4 the cells were harvested; the cell suspension was mixed with trypan blue and live cells were manually counted using a hemocytometer and dead cells were counted using a TC20 Automated cell counter (BioRad, USA). 1% Triton X was used as a positive control for cell death.

### Reactive oxygen species (ROS) production

HSFs were seeded at a density of 2.5×10⁴ cells/0.32cm² and were grown to confluence overnight. The cells were stained with 20uM DCFDA solution (2',7'-dichlorofluorescin diacetate) (DCFDA Cellular ROS Detection Assay Kit, Abcam, Cambridge, UK) and incubated for 45 min at 37°C protected from light exposure. Control cell medium (5% FBS, dissolved oxygen: 8.6 mg/L), oxygenated cell medium (5% FBS, dissolved oxygen: 21.6, 26.5, 34.9 mg/L) and positive control (500 µM HzOz, 5% FBS, dissolved oxygen: 8.6 mg/L) were added. The cells were incubated at room temperature. The fluorescence was read with an FLx800 plate reader (BioTek, Winooski, USA) with excitation wavelength at 485nm and emission wavelength 520 nm, 30 min after adding the treatments. The values were corrected for blank readings and presented as relative fluorescence (x10³). The experiments were repeated using 10% FBS.

### Statistics

All *in vitro* experiments were performed in triplicates and repeated at least 3 times. Results were analysed statistically in SPSS using two-way analysis with unpaired t-test or ANOVA with Bonferroni post hoc test comparing the average values of each experiment. Values of p < 0.05 were considered statistically significant.

### Results:

*In vitro* studies showed that ATP levels were significantly higher in human skin fibroblasts after four hourly stimulations of 33 mg/L dissolved oxygen compared to control (8 mg/L dissolved oxygen). The levels of ATP were 70.9 ± 20.5, 113.1 ± 10.1, and 191.6 ± 34.2 nM ATP/10⁵ live cells for control, 33 mg/L dissolved oxygen, and positive control, respectively (see Figure 5). Increasing the concentration of dissolved oxygen up to 50 mg/L did not change cell proliferation of HSF (see Figure 6), nor did it change the level of cell viability when compared to the control (11 mg/L dissolved oxygen) (see Figure 7). Increasing the concentration of dissolved oxygen to 34.9 mg/L did not significantly change the ROS production after 30 min in HSF when compared to control (8.6 mg/L dissolved oxygen). The relative fluorescence (x10³) was 13.5 ± 3.9, 9.3 ± 3.4, 15.2 ± 4.5, 8.9 ± 2.9, and 187.6 ± 18.0 for control, 21.6 mg/L dissolved oxygen, 26.5 mg/L dissolved oxygen, 34.9 mg/L dissolved oxygen and positive control, respectively (see Figure 8).

### Conclusions:

The present studies show that treatment of cells with elevated dissolved oxygen levels significantly increased ATP production without affecting proliferation, cell viability, or oxidative stress *in vitro.*
4 hourly stimulations of 33 mg/L dissolved oxygen resulted in increased ATP levels in HSFs when compared to the control. This indicates an advantageous effect of dissolved oxygen on the energy levels of human skin cells, thus facilitating increased wound healing. The results presented herein do not show any significant change in proliferation or viablility after treating the HSF with dissolved oxygen levels up to 50 mg/L for four days, indicating that topical treatment with dissolved oxygen up to 50 mg/L is safe and does not induce cellular toxicity.

In the studies described herein, no significant increase in ROS was found after 30 min in HSFs stimulated with increased levels of dissolved oxygen when compared to the control, either when using 5% or 10% FBS. Thus, increasing the dissolved oxygen levels up to 34.9 mg/L does not induce damaging levels of ROS. This is further reflected in results from quantification of cell death after daily stimulations with highly oxygenated cell medium (up to 50 mg/L), where no difference in toxicity was observed after 4 days when compared to the control.

The oxygenated water can be used in the production of dressings with various dissolved oxygen concentrations and pH. With proper monitoring of a wound, it is expected that the dressings can optimize chronic wound treatment.

### Example 7 - Preparation of thermogelling formulation

An oxygenated thermogelling formulation was prepared by oxygenation of a poloxamer-containing composition.

### Materials:

Poloxamers: Kolliphor P407 (Sigma Aldrich)
   Kolliphor P188 (Sigma Aldrich)
Buffer: Tri-sodium citrate (anhydrous)
   NaCl
   Citric acid
   MilliQ H₂O
   pH 5.1

### Methods:

### Preparation of 10 mM citrate buffer:

Base (10 mM tri-sodium citrate/140mM NaCl): 11.76 g of tri-sodium citrate (anhydrous) and 32.73 g NaCl were dissolved in MilliQ H₂O to a final volume of 4000 ml.
Acid (10 mM citric acid/140mM NaCl): 1.92 g citric acid and 8.18 g NaCl were dissolved in MilliQ H₂O to a final volume of 1000 ml.
3000ml of base was mixed with 750 ml of acid. pH was adjusted to 5.1 using the acid or base and the buffer was placed in a fridge or cold room.

### Preparation of formulation:

Cold buffer equal to 80 wt.% of the final volume of the formulation was placed in a wide flask and stirred with a magnetic stir bar. Slowly the poloxamers were added to buffer whilst stirring to produce a solution containing 18.5 wt.% Kolliphor P407 and 1.5 wt.% Kolliphor P188. The solution was mixed on ice in a cold room overnight or until the solution was completely clear and lump free.

### Oxygenation of formulation:

The poloxamer-containing formulation was oxygenated by passing it through the device described in WO 2016/071691. The O₂ pressure was set to 4.1 bar (60 psi). The formulation was passed through the device once at a flow rate of 100 ml/min O₂.

### Testing:

Samples of the oxygenated formulation were taken for Winkler titration according to the protocol described in Example 5, and for gelling and pH measurements. Gelling ability included gelling of both 100 µl and 1 ml sampels to compare gelling of large and small volumes of the formulation on a 35°C surface.

### Results:

Passing the formulation through the oxygenation device once with 100 ml/min O₂ did not lead to a significant foam build-up, even without the addition of foam reducing agents. The average dissolved oxygen content measured with Winkler titration was 8.2 ± 1.1 (n=4), 10.4 ± 5.9 (n=4), and 36.1± 3.8 mg/L (n=4) for the baseline, no oxygen (single pass), and oxygenated (single pass), respectively. The oxygenated sample had dissolved oxygen values significantly higher than baseline.

The pH did not change when circulating the formulation through the device without/with oxygen, with an average pH of 5.47 ± 0.04 and 5.47 ± 0.05, respectively. The gelling of 100 µl formulation and 1 ml formulation applied to a 35°C surface took approximately 3 min and 5 min, respectively, independent of the addition of oxygen.

### Conclusions:

Oxygenation of the poloxamer-containing formulation up to 36.1 ± 3.8 mg/L was achieved after a single pass through the oxygenation device and without changing the pH or the thermogelling properties. A single pass did not result in foam build-up even without the addition of foam reducing agents.

### Example 8 - Preparation of thermogelling formulation

### Method:

The method of Example 7 was repeated subject to the following changes:
EX CELL foam reducer (simethicone emulsion, Sigma Aldrich) was added to the buffer or to the formulation immediately prior to oxygenation.

The formulation was passed through the oxygenation device either once or twice at a flow rate of 200 ml/min O₂.

### Results:

The average dissolved oxygen content measured with Winkler titration was 11.0 ± 1.7 (n=3), 12.4 ± 2.8 (n=3), 46.8 ± 5.7 mg/L (n=3), and 42.7 ± 3.2 mg/L (n=3) for the baseline, no oxygen, oxygenated (single pass) and oxygenated (multiple passes), respectively. The oxygenated samples had dissolved oxygen values significantly higher than baseline.

With a starting pH of 5.1 in the buffer, the pH at baseline in the final formulation was 5.54 ± 0.04. This did not change after circulating the formulation through the device without/with oxygen. The gelling of 100 µl formulation and 1 ml formulation applied to a 35°C surface took approximately 3 min and 5 min, respectively, independent of the addition of oxygen.

### Conclusions:

18.5 wt.%/1.5 wt.% Kolliphor 407/188 in a 10 mM citrate buffer has thermogelling properties and can be regulated to a pH of 5.5. Oxygenation of the formulation up to 46.8 ± 5.7 mg/L was achieved without changing the pH or the thermogelling properties. Circulating the formulation several times through the device did not increase the dissolved oxygen levels, but increased the foam-build up.

## Claims

1. A liquid formulation comprising at least 20 mg/L dissolved oxygen and an *in-situ* gelling agent which is capable of forming a gel upon contact with a body surface or body tissues and which comprises one or more poloxamers, and wherein said gel is capable of releasing a therapeutically effective amount of said dissolved oxygen.

2. A formulation as claimed in claim 1 which comprises up to 100 mg/L dissolved oxygen, e.g. from 25 to 70 mg/L dissolved oxygen.

3. A formulation as claimed in claim 1, wherein said *in-situ* gelling agent comprises at least one poloxamer having a thermogelling temperature above 25°C, e.g. in the range from 25 to 37°C, preferably wherein said *in-situ* gelling agent comprises one or more poloxamers selected from Poloxamer 407, Poloxamer 188, Poloxamer 338, Poloxamer 124, and Poloxamer 237, more preferably wherein said *in-situ* gelling agent comprises Poloxamer 407.

4. A formulation as claimed in any one of claims 1 to 3 which comprises a blend of poloxamers, preferably wherein said blend comprises two poloxamers in a ratio of from 5:1 to 50:1, preferably from 10:1 to 25:1, e.g. from 10:1 to 15:1, for example wherein said blend comprises Poloxamer 407 and Poloxamer 188, or Poloxamer 407 and Poloxamer 124.

5. A formulation as claimed in any one of claims 1 to 4, wherein said poloxamer or blend of poloxamers is present in an amount in the range of from 10 to 30 wt.%, preferably 15 to 25 wt.%, e.g. 16 to 20 wt.% (based on the weight of the formulation).

6. A formulation as claimed in any one of claims 1 to 5 which further comprises:
(i) one or more thickening agents selected from the following: Carbopol, cellulose derivatives (e.g. hydroxypropyl cellulose or hydroxypropyl methylcellulose), carrageenans, gelatin, pectin, hydrocolloids, alginates, hydrogels, polyurethane, collagen, chitosan, and hyaluronic acid; and/or
(ii) one or more bioadhesives, e.g. one or more mucoadhesive polymers; and/or
(iii) one or more active substances selected from the group consisting of:
antibacterial agents, antifungal agents, antiviral agents, antibiotics, growth factors, cytokines, chemokines, nucleic acids, vitamins, minerals, anaesthetics, antiinflammatory agents, moisturizers, extracellular matrix proteins, enzymes, stem cells from plants, extracts from eggs and eggshells, botanical extracts, fatty acids, and skin penetration enhancers, preferably wherein said antibacterial agent is selected from the group consisting of: alcohols, chlorine, peroxides, aldehydes, triclosan, triclocarban, benzalkonium chloride, linezolid, quinupristin-dalfopristin, daptomycin, oritavancin and dalbavancin, quinolones, and moxifloxacin; and/or
(iv) at least one active agent selected from the group consisting of retinoids (e.g. vitamin A, acitretin, isotretinoin, tretinoin and tazarotene); peroxides (e.g. benzoyl peroxide); antibiotics (e.g. tetracycline, clindamycin, erythromycin, metronidazole, sulfacetamide, doxycycline, oxytetracycline, minocycline, and trimethoprim); hormones (e.g. co-cyprindiol); azelaic acid and derivatives thereof; adapalene; nicotinamide; salicylic acid; corticosteroids, vitamin D and derivatives thereof; antralin, and calcineurin inhibitors; and/or
(v) at least one wound healing agent, e.g. collagen or chitosan.

7. A formulation as claimed in any one of the preceding claims, wherein the oxygen present in the formulation is dissolved in an aqueous medium which is physiologically tolerable, for example a physiological salt solution (e.g. saline) or water, preferably wherein said formulation comprises from 50 to 99 wt.% water, more preferably from 80 to 99 wt.% water.

8. A formulation as claimed in any one of the preceding claims which comprises an oxygenated liquid (e.g. physiological saline or water) obtainable by (e.g. obtained by) a process comprising the following steps:
• introducing a pressurized liquid into a piping network to form a flow stream;
• injecting gaseous oxygen into the flow stream to produce a mixture of liquid and oxygen bubbles,
• providing a linear flow accelerator including a venturi; and
• passing the flowing mixture of liquid and gaseous oxygen bubbles through the linear flow accelerator to accelerate the flowing mixture and to subsequently decelerate the flowing mixture to subsonic speed to break up the gaseous oxygen bubbles.

9. A formulation as claimed in any one of the preceding claims which is a cosmetic formulation and which comprises one or more cosmetically active ingredients selected from the group consisting of peptides, amino acids, hyaluronic acid, hydroxy acids, vitamins or derivatives thereof, retinoids, ceramides, carbamide (urea) and coenzyme Q10.

10. A formulation as claimed in any one of the preceding claims which comprises:
(i) one or more components which maintain a buffered pH, which maintain osmolality in a range suitable for application to a body surface or body tissues, or which maintain stability of the composition; and/or
(ii) one or more components selected from the group consisting of: buffers, pH adjusting agents (e.g. sodium hydroxide or hydrochloric acid), osmolality adjusting agents, preservatives (e.g. anti-microbial agents), anti-oxidants, gel forming agents (e.g. Carbopols or cellulose derivatives), fragrances, and coloring agents, preferably sufficient buffer to provide a pH in the range from 2 to 7, preferably 5 to 5.5, e.g. about 5.1 to about 5.5.

11. An oxygen-releasing gel obtainable by allowing the liquid formulation as claimed in any one of claims 1 to 10 to gel.

12. A formulation or oxygen-releasing gel as claimed in any one of claims 1 to 11 for use in medicine or for use as a medicament, preferably for use in the treatment of a compromised tissue, for example a wound (acute or chronic), a burn, a skin disorder (e.g. psoriasis, acne, rosacea, or other dermatological condition such as atopic dermatitis), skin sores, or tissue necrosis, more preferably for use in the treatment of bacterial or fungal infections of the skin, for example cellulitis, infections in chronic wounds, gas gangrene, necrotizing fasciitis (infection by enterococcus, enterobacteriacea, clostridium, B. fragilis, streptococcus, pyogenis), or a fungal infection associated with mucorales or aspergillus, or for use in the prevention or treatment of a bacterial biofilm on a body surface, preferably on an external body surface, e.g. on the skin.

13. A delivery device (e.g. a wound dressing or bandage) having incorporated therein a liquid formulation as claimed in any one of claims 1 to 10 or an oxygen-releasing gel as claimed in claim 11.

14. A kit for use in the treatment of a compromised body surface or body tissue of a mammalian subject (e.g. a human), the kit comprising:
(a) a sealed container or package containing a liquid formulation as claimed in any one of claims 1 to 10;
(b) a delivery device, e.g. a wound dressing or bandage; and optionally
(c) instructions for use of components (a) and (b) in the topical treatment of a compromised tissue.

15. A method of cosmetic treatment performed on a mammalian subject (e.g. a human), said method comprising the step of administering to the surface of the skin of said subject a liquid formulation as claimed in any one of claims 1 to 10, preferably a method for improving or otherwise enhancing the appearance of the skin, for example in softening the skin, or in reducing any one of the following: hyper-pigmentation, roughness, dryness, fine lines and wrinkles of the skin.

## Patentansprüche

1. Flüssige Formulierung, umfassend mindestens 20 mg/l gelösten Sauerstoff und ein In-situ-Geliermittel, das in der Lage ist, bei Kontakt mit einer Körperoberfläche oder Körpergeweben ein Gel zu bilden und das ein oder mehrere Poloxamere umfasst, und wobei das Gel in der Lage ist, eine therapeutisch wirksame Menge des gelösten Sauerstoffs freizusetzen.

2. Formulierung nach Anspruch 1, die bis zu 100 mg/l gelösten Sauerstoff umfasst, z. B. von 25 bis 70 mg/l gelösten Sauerstoff.

3. Formulierung nach Anspruch 1, wobei das In-situ-Geliermittel mindestens ein Poloxamer umfasst mit einer Thermogelierungstemperatur oberhalb von 25 °C, z. B. im Bereich von 25 bis 37 °C, vorzugsweise wobei das In-situ-Geliermittel ein oder mehrere Poloxamere umfasst, ausgewählt aus Poloxamer 407, Poloxamer 188, Poloxamer 338, Poloxamer 124 und Poloxamer 237, mehr bevorzugt wobei das In-situ-Geliermittel Poloxamer 407 umfasst.

4. Formulierung nach einem der Ansprüche 1 bis 3, die ein Gemisch von Poloxameren umfasst, vorzugsweise wobei das Gemisch zwei Poloxamere in einem Verhältnis von 5 : 1 bis 50 : 1, vorzugsweise von 10 : 1 bis 25 : 1, z. B. 10 : 1 bis 15 : 1 umfasst, zum Beispiel wobei das Gemisch Poloxamer 407 und Poloxamer 188 oder Poloxamer 407 und Poloxamer 124 umfasst.

5. Formulierung nach einem der Ansprüche 1 bis 4, wobei das Poloxamer oder Gemisch von Poloxameren in einer Menge im Bereich von 10 bis 30 Gew.-%, vorzugsweise 15 bis 25 Gew.-%, z. B. 16 bis 20 Gew.-% (bezogen auf das Gewicht der Formulierung) vorhanden ist.

6. Formulierung nach einem der Ansprüche 1 bis 5, die weiter umfasst:
(i) ein oder mehrere Verdickungsmittel, ausgewählt aus dem Folgenden: Carbopol, Cellulosederivativen (z. B. Hydroxypropylcellulose oder Hydroxypropylmethylcellulose), Carrageenanen, Gelatine, Pectin, Hydrocolloiden, Alginaten, Hydrogelen, Polyurethan, Kollagen, Chitosan und Hyaluronsäure; und/oder
(ii) ein oder mehrere Bioadhäsive, z. B. ein oder mehrere mukoadhäsive Polymere; und/oder
(iii) einen oder mehrere Wirkstoffe, ausgewählt aus der Gruppe bestehend aus:
antibakteriellen Mitteln, antimykotischen Mitteln, antiviralen Mitteln, Antibiotika, Wachstumsfaktoren, Zytokinen, Chemokinen, Nukleinsäuren, Vitaminen, Mineralien, Anästhetika, entzündungshemmenden Mitteln, Feuchtigkeitsspendern, extrazellulären Matrixproteinen, Enzymen, Stammzellen aus Pflanzen, Extrakten aus Eiern und Eierschalen, botanischen Extrakten, Fettsäuren und Hautpenetrationsförderern, vorzugsweise wobei das antibakterielle Mittel ausgewählt ist aus der Gruppe, bestehend aus: Alkoholen, Chlor, Peroxiden, Aldehyden, Triclosan, Triclocarban, Benzalkoniumchlorid, Linezolid, Quinupristin-Dalfopristin, Daptomycin, Oritavancin und Dalbavancin, Chinolonen und Moxifloxacin; und/oder
(iv) mindestens ein Aktivmittel, ausgewählt aus der Gruppe, bestehend aus Retinoiden (z. B. Vitamin A, Acitretin, Isotretinoin, Tretinoin und Tazaroten); Peroxiden (z. B. Benzoylperoxid); Antibiotika (z. B. Tetrazyklin, Clindamycin, Erythromycin, Metronidazol, Sulfacetamid, Doxycyclin, Oxytetracyclin, Minocyclin und Trimethoprim); Hormonen (z. B. Co-Cyprindiol); Azelainsäure und Derivaten davon; Adapalen; Nicotinamid; Salicylsäure, Kortikosteroiden, Vitamin D und Derivaten davon; Antralin und Calcineurininhibitoren; und/oder
(v) mindestens einem Wundheilungsmittel, z. B. Kollagen oder Chitosan.

7. Formulierung nach einem der vorstehenden Ansprüche, wobei der in der Formulierung vorhandene Sauerstoff in einem wässrigen Medium gelöst ist, das physiologisch verträglich ist, zum Beispiel einer physiologischen Salzlösung (z. B. Kochsalzlösung) oder Wasser, vorzugsweise wobei die Formulierung von 50 bis 99 Gew.-% Wasser, mehr bevorzugt von 80 bis 99 Gew.-% Wasser umfasst.

8. Formulierung nach einem der vorstehenden Ansprüche, die eine sauerstoffangereicherte Flüssigkeit (z. B. physiologische Kochsalzlösung oder Wasser) umfasst, die durch einen Prozess erhältlich ist (z. B. erhalten wird durch), der die folgenden Schritte umfasst:
- Einleiten einer druckbeaufschlagten Flüssigkeit in ein Rohrleitungsnetzwerk, um einen Durchflussstrom zu bilden;
- Injizieren gasförmigen Sauerstoffs in den Durchflussstrom, um eine Mischung von Flüssigkeit und Sauerstoffblasen zu produzieren,
- Bereitstellen eines Linearströmungsbeschleunigers, einschließlich eines Venturis; und
- Leiten der fließenden Mischung von Flüssigkeit und gasförmigen Sauerstoffblasen durch den Linearströmungsbeschleuniger hindurch, um die fließende Mischung zu beschleunigen und nachfolgend die fließende Mischung auf Unterschallgeschwindigkeit zu entschleunigen, um die gasförmigen Sauerstoffbläschen zu brechen.

9. Formulierung nach einem der vorstehenden Ansprüche, die eine kosmetische Formulierung ist und die einen oder mehrere kosmetische Wirkstoffe umfasst, ausgewählt aus der Gruppe, bestehend aus Peptiden, Aminosäuren, Hyaluronsäure, Hydroxysäuren, Vitaminen oder Derivaten davon, Retinoiden, Ceramiden, Carbamid (Harnstoff) und Coenzym Q10.

10. Formulierung nach einem der vorstehenden Ansprüche, die umfasst:
(i) eine oder mehrere Komponenten, die einen gepufferten pH-Wert aufrecht erhalten, die Osmolalität in einem Bereich aufrecht erhalten, der zur Auftragung auf eine Körperoberfläche oder Körpergewebe geeignet ist, oder der Stabilität der Zusammensetzung aufrecht erhält; und/oder
(ii) eine oder mehrere Komponenten, ausgewählt aus der Gruppe, bestehend aus Puffern, pH-Wert-Einstellungsmitteln (z. B. Natriumhydroxid oder Salzsäure), Osmolalität-Einstellungsmitteln, Konservierungsstoffen (z. B. antimikrobielle Mittel), Antioxidanzien, gelbildenden Mitteln (z. B. Carbopole oder Cellulosederivate), Duftstoffen und Farbmitteln, vorzugsweise ausreichend Puffer, um einen pH-Wert im Bereich von 2 bis 7, vorzugsweise 5 bis 5,5, z. B. etwa 5,1 bis etwa 5,5 bereitzustellen.

11. Sauerstofffreisetzendes Gel, erhältlich durch Gelierenlassen der flüssigen Formulierung nach einem der Ansprüche 1 bis 10.

12. Formulierung oder sauerstofffreisetzendes Gel nach einem der Ansprüche 1 bis 11 zur Verwendung in Medizin oder zur Verwendung als ein Medikament, vorzugsweise zur Verwendung bei der Behandlung eines geschädigten Gewebes, zum Beispiel einer Wunde (akut oder chronisch), einer Verbrennung, einer Hautstörung (z. B. Psoriasis, Akne, Rosacea oder weiteren dermatologischen Zustands wie atopischer Dermatitis), Hautwunden oder Gewebenekrose, mehr bevorzugt zur Verwendung bei der Behandlung von bakteriellen oder mykotischen Infektionen der Haut, zum Beispiel Cellulitis, Infektionen in chronischen Wunden, Gasbrand, nekrotisierender Fasziitis (Infektion durch Entercoccus,
Enterobacteriacea, Clostridium, B. fragilis, Streptococcus, Pyogenis), oder einer mykotischen Infektion, die mit Mucorales oder Aspergillus assoziiert ist, oder zur Verwendung bei der Prävention oder Behandlung eines bakteriellen Biofilms auf einer Körperoberfläche, vorzugsweise auf einer externen Körperoberfläche, z. B. auf der Haut.

13. Abgabevorrichtung (z. B. ein(e) Wundverband oder -bandage), das darin eingearbeitet eine flüssige Formulierung nach einem der Ansprüche 1 bis 10 oder ein sauerstofffreisetzendes Gel nach Anspruch 11 aufweist.

14. Kit zur Verwendung bei der Behandlung einer geschädigten Körperoberfläche oder eines geschädigten Körpergewebes eines Säugetiersubjekts (z. B. eines Menschen), wobei das Kit umfasst:
(a) einen abgedichteten Behälter oder eine abgedichtete Verpackung, die eine flüssige Formulierung nach einem der Ansprüche 1 bis 10 enthält;
(b) eine Abgabevorrichtung, z. B. ein(e) Wundverband oder -bandage; und wahlweise
(c) Anweisungen zur Verwendung von Komponenten (a) und (b) bei der topischen Behandlung eines geschädigten Gewebes.

15. Verfahren zur kosmetischen Behandlung, durchgeführt an einem Säugetiersubjekt (z. B. einem Menschen), wobei das Verfahren den Schritt des Verabreichens einer flüssigen Formulierung nach einem der Ansprüche 1 bis 10 an die Oberfläche der Haut des Subjekts umfasst, vorzugsweise ein Verfahren zum Verbessern oder anderweitigen Fördern des Erscheinungsbilds der Haut, zum Beispiel durch Erweichen der Haut, oder durch Verringern eines beliebigen des Folgenden: Hyperpigmentierung, Rauheit, Trockenheit, feine Linien und Falten der Haut.

## Revendications

1. Formulation liquide comprenant au moins 20 mg/l d'oxygène dissous et un agent gélifiant *in situ* qui permet de former un gel au contact avec une surface corporelle ou de tissus corporels et qui comprend un ou plusieurs poloxamères et dans laquelle ledit gel permet de libérer une quantité thérapeutiquement efficace dudit oxygène dissous.

2. Formulation selon la revendication 1, qui comprend jusqu'à 100 mg/l d'oxygène dissous, par exemple de 25 à 70 mg/l d'oxygène dissous.

3. Formulation selon la revendication 1, dans laquelle ledit agent gélifiant *in situ* comprend au moins un poloxamère ayant une température de thermogélification supérieure à 25 °C, par exemple dans la plage de 25 à 37 °C, de préférence dans laquelle ledit agent gélifiant in situ comprend un ou plusieurs poloxamères sélectionnés parmi le poloxamère 407, le poloxamère 188, le poloxamère 338, le poloxamère 124 et le poloxamère 237, plus préférentiellement dans laquelle ledit agent gélifiant comprend le poloxamère 407.

4. Formulation selon l'une quelconque des revendications 1 à 3, qui comprend un mélange de poloxamères, de préférence dans laquelle ledit mélange comprend deux poloxamères dans un rapport de 5:1 à 50:1, de préférence de10:1 à 25:1, par exemple de 10:1 à 15:1, par exemple dans laquelle ledit mélange comprend le poloxamère 407 et le poloxamère 188 ou le poloxamère 407 et le poloxamère 124.

5. Formulation selon l'une quelconque des revendications 1 à 4, dans laquelle ledit poloxamère ou ledit mélange de poloxamères est présent en une quantité dans la plage de 10 à 30 % en poids, de préférence 15 à 25 % en poids, par exemple 16 à 20 % en poids (sur la base du poids de la formulation).

6. Formulation selon l'une quelconque des revendications 1 à 5, qui comprend en outre :
(i) un ou plusieurs agents épaississants sélectionnés parmi les éléments suivants : le carbopol, des dérivés de cellulose (par exemple, l'hydroxypropyl cellulose ou l'hydroxypropyl méthylcellulose), des carragénines, une gélatine, une pectine, des hydrocolloïdes, des alginates, des hydrogels, un polyuréthane, le collagène, le chitosane et l'acide hyaluronique ; et/ou
(ii) un ou plusieurs bioadhésifs, par exemple un ou plusieurs polymères muco-adhésifs ; et/ou
(iii) une ou plusieurs substances actives sélectionnées dans le groupe constitué par : des agents antibactériens, des agents antifongiques, des agents antiviraux, des antibiotiques, des facteurs de croissance, des cytokines, des chémokines, des acides nucléiques, des vitamines, des minéraux, des anesthésiques, des agents anti-inflammatoires, des hydratants, des protéines de la matrice extracellulaire, des enzymes, des cellules souches provenant de plantes, des extraits d'oeufs et de coquilles d'oeuf, des extraits botaniques, des acides gras et des activateurs de pénétration de la peau, de préférence, dans laquelle ledit agent antibactérien est sélectionné dans le groupe constitué par : des alcools, le chlore, des peroxydes, des aldéhydes, triclosan, le triclocarban, le chlorure de benzalkonium, le linézolide, la quinupristine-dalfopristine, la daptomycine, l'oritavancine et la dalbavancine, des quinolones et la moxifloxacine ; et/ou
(iv) au moins un agent actif sélectionné dans le groupe constitué par des rétinoïdes (par exemple, la vitamine A, l'acitrétine, l'isotrétinoïne, la tretinoïne et le tazarotène) ; des peroxydes (par exemple, le peroxyde de benzoyle) ; des antibiotiques (par exemple, la tétracycline, la clindamycine, l'érythromycine, le métronidazole, le sulfacétamide, la doxycycline, l'oxytétracycline, la minocycline et le triméthoprime) ; des hormones (par exemple, le co-cyprindiol) ; l'acide azélaïque et des dérivés celui-ci ; l'adapalène ; la nicotinamide ; l'acide salicylique ; des corticosteroïdes, la vitamine D et des dérivés de ceux-ci ; l'antraline et des inhibiteurs de la calcineurine ; et/ou
(v) au moins un agent de cicatrisation de blessure, par exemple, le collagène ou le chitosane.

7. Formulation selon l'une quelconque des revendications précédentes, dans laquelle l'oxygène présent dans la formulation est dissous dans un milieu aqueux qui est physiologiquement tolérable, par exemple, une solution de sel physiologique (par exemple, saline) ou de l'eau, de préférence dans laquelle ladite formulation comprend de 50 à 99 % en poids d'eau, plus préférentiellement de 80 à 99 % en poids d'eau.

8. Formulation selon l'une quelconque des revendications précédentes, qui comprend un liquide oxygéné (par exemple, du sérum physiologique ou de l'eau) pouvant être obtenu par (par exemple, obtenu par) un processus comprenant les étapes suivantes :
- l'introduction d'un liquide sous pression dans un réseau de tuyauterie pour former un flux d'écoulement ;
- l'injection de l'oxygène gazeux dans le flux d'écoulement pour produire un mélange de liquide et de bulles d'oxygène,
- la fourniture d'un accélérateur d'écoulement linéaire incluant un venturi ; et
- le passage du mélange fluide de liquide et de bulles d'oxygène gazeux à travers l'accélérateur d'écoulement linéaire pour accélérer le mélange fluide et pour décélérer, par la suite, le mélange fluide à une vitesse subsonique pour casser les bulles d'oxygène gazeux.

9. Formulation selon l'une quelconque des revendications précédentes, qui est une formulation cosmétique et qui comprend un ou plusieurs ingrédients cosmétiquement actifs sélectionnés dans le groupe constitué par des peptides, des acides aminés, l'acide hyaluronique, des acides hydroxy, des vitamines ou des dérivés de celles-ci, des rétinoïdes, des céramides, le carbamide (l'urée) et la coenzyme Q10.

10. Formulation selon l'une quelconque des revendications précédentes, qui comprend :
(i) un ou plusieurs composants qui maintiennent un pH tamponné, qui maintiennent l'osmolalité dans une plage appropriée pour une application à une surface corporelle ou à des tissus corporels, ou qui maintiennent la stabilité de la composition ; et/ou
(ii) un ou plusieurs composants sélectionnés dans le groupe constitué par des tampons, des agents d'ajustement du pH (par exemple, l'hydroxyde de sodium ou l'acide chlorhydrique), des agents d'ajustement de l'osmolalité, des conservateurs (par exemple, des agents antimicrobiens), des anti-oxydants, des agents de formation de gel (par exemple, des Carbopols ou des dérivés de cellulose), des fragrances et des colorants, de préférence un tampon suffisant pour fournir un pH dans la plage de 2 à 7, de préférence de 5 à 5,5, par exemple, d'environ 5,1 à environ 5,5.

11. Gel de libération d'oxygène pouvant être obtenu en permettant la formulation liquide selon l'une quelconque des revendications 1 à 10 à un gel.

12. Formulation, ou gel de libération d'oxygène, selon l'une quelconque des revendications 1 à 11 destinée à être utilisée en médecine ou destinée à être utilisée comme médicament, de préférence destinée à être utilisée dans le traitement d'un tissu fragilisé, par exemple, une plaie (aiguë ou chronique), une brûlure, une affection cutanée (par exemple, le psoriasis, l'acné, la rosacée ou une autre maladie dermatologique telle que la dermatite atopique), des lésions cutanées ou une nécrose tissulaire, plus préférentiellement destinée à être utilisée dans le traitement d'infections bactériennes ou fongiques de la peau, par exemple, la cellulite, des infections de plaies chroniques, la gangrène gazeuse, la fasciite nécrosante (une infection à entérocoques,
à entérobactéries, le Clostridium, B. fragilis, un streptocoque, pyogenis), ou une infection fongique associée à des mucorales ou à aspergillus, ou destinée à être utilisée dans la prévention ou le traitement d'un biofilm bactérien sur une surface corporelle, de préférence sur une surface corporelle externe, par exemple sur la peau.

13. Dispositif d'administration (par exemple, des pansements de plaies ou des bandages) dans lequel est incorporée une formulation liquide selon l'une quelconque des revendications 1 à 10 ou un gel de libération d'oxygène selon la revendication 11.

14. Kit destiné à être utilisé dans le traitement d'une surface corporelle affaiblie ou un tissu corporel affaibli d'un sujet mammifère (par exemple, un être humain), le kit comprenant :
(a) un récipient, ou un boîtier, scellé contenant une formulation liquide selon l'une quelconque des revendications 1 à 10 ;
(b) un dispositif d'administration, par exemple, un pansement de plaie ou un bandage ; et facultativement
(c) des instructions pour l'utilisation de composants (a) et (b) dans le traitement topique d'un tissu affaibli.

15. Procédé de traitement cosmétique réalisé sur un sujet mammifère (par exemple, un être humain), ledit procédé comprenant l'étape d'administration, à la surface de la peau dudit sujet, d'une formulation liquide selon l'une quelconque des revendications 1 à 10, de préférence procédé pour bonifier ou, sinon, améliorer l'apparence de la peau, par exemple, en adoucissant la peau ou en réduisant l'un quelconque des caractéristiques suivantes : une hyperpigmentation, la rugosité, la sécheresse, les ridules et les rides de la peau.
